# EUROPEAN PATENT APPLICATION

(11) **EP 2 371 219 A1**
(43) Date of publication of application: **05.10.2011**
(21) Application number: 10158922.4
(22) Date of filing: 01.04.2010
(51) Int. Cl.: A01N 43/38, A01N 43/90, A01P 13/00, A61K 31/4035, A61K 31/407, C07D 209/50, C07D 209/68, C07D 487/04

(54) **Herbicidal acylhydrazides**

(71) Applicant: BASF SE, 67056 Ludwigshafen (DE)
(72) Inventor: The designation of the inventor has not yet been filed

(57) **Abstract**

The present invention relates to a method for controlling undesired vegetation by application of acylhydrazides of the general formula I 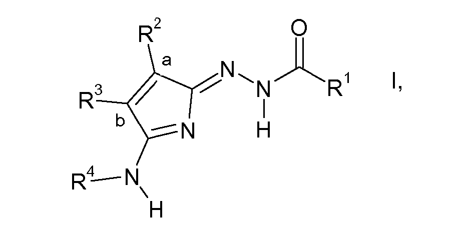 wherein the variables are defined according to the description, processes and intermediates for preparing the acylhydrazides of the formula I, compositions comprising them and their use as herbicides, i.e. for controlling harmful plants, a method for controlling unwanted vegetation which comprises allowing a herbicidal effective amount of at least one acylhydrazide of the formula I to act on plants, their seed and/or their habitat as well as to their use as pharmaceutical active compound and a method of treating or preventing infections by application of the acylhydrazides of the formula I.

## Description

The present invention relates to a method for controlling undesired vegetation by application of acylhydrazides of the general formula I defined below.

Hordiyenko et al, Eur. J. Org. Chem. 2006, 2833-2842 describes structurally similar compounds and their amide-type isomerism, but does not mention nor any herbicidal neither any pharmaceutical activity or use of these compounds for treating or preventing parasitic and/or bacterial infections.

Surprisingly it has now been found that acylhydrazides of formula I effectively control undesired vegetation and are also useful for treating or preventing parasitic and/or bacterial infections.

The herbicidal properties of known compounds with regard to the harmful plants are not always entirely satisfactory. Furthermore there is a need for novel compounds and compositions for use in treating or preventing parasitic and/or bacterial infections.

Accordingly, the present invention provides a method of controlling undesired vegetation, which comprises allowing an herbicidal active amount of at least one acylhydrazide of formula I 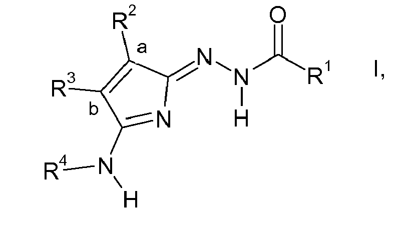 wherein the variables are as defined below:
R¹ is a five- or six-membered monocyclic or eight- to ten-membered bicyclic heteroaryl having one to three nitrogen atoms, one or two nitrogen atoms and one sulfur atom, one nitrogen and one oxygen atom, one oxygen atom, or one sulfur atom, which may be partially or fully halogenated and/or may be substituted by one to three substituents selected from the group consisting of cyano, nitro, C₁-C₆-alkyl, C₃-C₆-cycloalkyl, C₁-C₆-haloalkyl, C₂-C₆-alkenyl, C₂-C₆-alkynyl, hydroxy, C₁-C₆-alkoxy, C₃-C₆-cycloalkoxy, C₁-C₆-haloalkoxy, C₃-C₆-alkenyloxy, C₃-C₆-alkynyloxy, C₁-C₆-alkylthio, C₁-C₆-alkylsulfinyl, C₁-C₆-alkylsulfonyl, amino, C₁-C₆-alkylamino, di(C₁-C₆)alkylamino, C₁-C₆-alkylsulfonylamino, N(C₁-C₆-alkylsulfonyl)-N(C₁-C₆-alkyl)amino, five- or six-membered heterocyclyl having one or two nitrogen atoms, one nitrogen and one oxygen atom or one nitrogen and one sulfur atom, or phenyl, which itself may be partially or fully halogenated and/or may be substituted by one to three substituents selected from of the group consisting of C₁-C₆-alkyl, C₁-C₆-haloalkyl and C₁-C₆-alkoxy;
R² and R³ together with the carbon atoms (a) and (b) to which they are attached form a five- or six-membered monocyclic or eight- to ten-membered bicyclic cyclus,
   which is partially unsaturated or aromatic,
   which may contain one to three nitrogen atoms, one or two nitrogen atoms and one sulfur atom, one nitrogen and one oxygen atom, one or two sulfur atoms, or one or two oxygen atoms; and
   which may be partially or fully halogenated and/or may be substituted by one to three substituents selected from the group consisting of cyano, nitro, C₁-C₆-alkyl, C₃-C₆-cycloalkyl, C₁-C₆-haloalkyl, C₁-C₆-hydroxyalkyl, C₁-C₆-alkoxy-C₁-C₆-alkyl, C₁-C₆-haloalkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₂-C₆-alkenyl, C₂-C₆-haloalkenyl, C₂-C₆-hydroxyalkenyl, C₁-C₆-alkoxy-C₂-Cs-alkenyl, C₁-C₆-haloalkoxy-C₂-C₆-alkenyl, amino-C₂-C₆-alkenyl, (C₁-C₆-alkyl)amino-C₂-C₆-alkenyl, di(C₁-C₆-alkyl)amino-C₂-C₆-alkenyl, C₂-C₆-alkynyl, C₂-C₆-haloalkynyl, C₂-C₆-hydroxyalkynyl, C₁-C₆-alkoxy-C₂-C₆-alkynyl, C₁-C₆-haloalkoxy-C₂-C₆-alkynyl, amino-C₂-C₆-alkynyl, (C₁-C₆-alkyl)amino-C₂-C₆-alkynyl, di(C₁-C₆-alkyl)amino-C₂-C₆-alkynyl, C₁-C₆-alkylcarbonyl, hydroxycarbonyl, C₁-C₆-alkoxycarbonyl, aminocarbonyl, (C₁-C₆-alkyl)aminocarbonyl, di(C₁-C₆-alkyl)aminocarbonyl, hydroxy, C₁-C₆-alkoxy, C₁-C₆-haloalkoxy, C₃-C₆-cycloalkoxy, C₃-C₆-alkenyloxy, C₃-C₆-alkynyloxy, C₁-C₆-alkylcarbonyloxy, C₁-C₆-hydroxyalkoxy, C₁-C₆-alkoxy-C₁-C₆-alkoxy, C₁-C₆-haloalkoxy-C₁-C₆alkoxy, amino-C₁-C₆-alkoxy, (C₁-C₆-alkyl)amino-C₁-C₆-alkoxy, di(C₁-C₆-alkyl)amino-C₁-C₆-alkoxy, C₁-C₆-alkylsulfonyloxy, (C₁-C₆-alkyl)aminocarbonyl(C₁-C₆-alkyl)oxy, di(C₁-C₆-alkyl)-aminocarbonyloxy, C₁-C₆-alkylthio, C₃-C₆-cycloalkylthio, C₃-C₆-alkenylthio, C₃-C₆-alkynylthio, C₁-C₆-hydroxyalkylthio, C₁-C₆-alkoxy-C₁-C₆-alkylthio, C₁-C₆-haloalkoxy-C₁-C₆-alkylthio, amino-C₁-C₆-alkylthio, (C₁-C₆-alkyl)amino-C₁-C₆-alkylthio, di(C₁-C₆-alkyl)amino-C₁-C₆-alkylthio, amino, C₁-C₆-alkylamino, di-(C₁-C₆-)alkylamino, C₃-C₆-cycloalkylamino, C₃-C₆-alkenylamino, C₃-C₆-alkynylamino, C₁-C₆-hydroxyalkylamino, C₁-C₆-alkoxy-C₁-C₆-alkylamino, C₁-C₆-haloalkoxy-C₁-C₆-alkylamino, amino-C₁-C₆-alkylamino, (C₁-C₆-alkyl)amino-C₁-C₆-alkylamino, di(C₁-C₆-alkyl)amino-C₁-C₆-alkylamino, C₁-C₆-alkylsulfonylamino, C₁-C₆-alkylsulfonyl(C₁-C₆-alkyl)amino, (C₁-C₆-alkyl)aminosulfonylamino, di(C₁-C₆-alkyl)aminosulfonylamino, formylamino, formyl(C₁-C₆-alkyl)amino, (C₁-C₆-alkyl)carbonylamino, N(C₁-C₆-alkylcarbonyl)-N(C₁-C₆-alkyl)amino, (C₁-C₆-alkyl)aminocarbonylamino, di(C₁-C₆-alkyl)aminocarbonylamino, (C₁-C₆-alkyl)aminocarbonyl(C₁-C₆-alkyl)amino, C₁-C₆-alkylsulfinyl, C₁-C₆-alkylsulfonyl, C₃-C₆-cycloalkylsulfonyl, C₃-C₆-alkenylsulfonyl, C₃-C₆-alkynylsulfonyl, C₁-C₆-hydroxyalkylsulfonyl, C₁-C₆-alkoxy-C₁-C₆-alkylsulfonyl, C₁-C₆-haloalkoxy-C₁-C₆-alkylsulfonyl, amino-C₁-C₆-alkylsulfonyl, (C₁-C₆-alkyl)-amino-C₁-C₆-alkylsulfonyl, di(C₁-C₆-alkyl)amino-C₁-C₆-alkylsulfonyl, aminosulfonyl, (C₁-C₆-alkyl)aminosulfonyl, di(C₁-C₆-alkyl)aminosulfonyl, phenyl, phenylcarbonyl, phenyloxy, phenylcarbonyloxy, phenylthio, phenylsulfinyl, phenylsulfonyl, phenylsulfonyloxy, phenylamino, N(phenyl)-N(C₁-C₆-alkyl)amino, phenylcarbonylamino, N-phenylcarbonyl-N(C₁-C₆-alkyl)amino,
   wherein each of the phenyl groups may be partially or fully halogenated and/or may carry one to three substituents selected from the group consisting of cyano, C₁-C₆-alkyl, C₁-C₆-haloalkyl, hydroxy, C₁-C₆-alkoxy, C₁-C₆-alkylcarbonyloxy, C₁-C₆-haloalkoxy, C₃-C₆-alkenyloxy, C₃-C₆-alkynyloxy, C₁-C₆-alkylthio, C₁-C₆-alkylsulfonyl; heterocyclyl, heterocyclyl-C₁-C₆-alkyl, heterocyclyl-C₂-C₆-alkenyl, heterocyclyl-C₂-C₆-alkynyl, heterocydyl-C₁-C₆-alkoxy, heterocyclyl-C₁-C₆-alkylthio, heterocydyl-C₁-C₆-alkylamino, heterocyclyl-C₁-C₆-alkylsulfonyl, heterocyclyloxy, heterocyclylthio, heterocyclylsulfinyl, heterocyclylsulfonyl, heterocyclylsulfonyloxy, heterocyclylcarbonyl, heterocyclylcarbonyloxy, heterocyclylamino, heterocydyl-[(C₁-C₆-alkyl)amino], heterocyclylcarbonylamino, heterocyclylcarbonyl[(C₁-C₆-alkyl)amino],
   wherein each of the heterocyclyl groups being three- to seven-membered and having one to three nitrogen atoms, one or two nitrogen atoms and one sulfur atom, one nitrogen atom and one oxygen atom, one oxygen atoms, one or two sulfur atoms, one oxygen atom, or one sulfur atom, and wherein each of the heterocyclyl groups may be partially or fully halogenated and/or may be substituted with one to three substituents selected from the group consisting of cyano, C₁-C₆-alkyl, C₁-C₆-haloalkyl, hydroxy, mercapto, C₁-C₆-alkoxy, C₁-C₆-alkylcarbonyloxy, C₁-C₆-haloalkoxy, C₃-C₆-alkenyloxy, C₃-C₆-alkynyloxy, C₁-C₆-alkylthio, C₁-C₆-alkylsulfonyl;
and
R⁴ is hydrogen, formyl or C₁-C₆-alkylcarbonyl;
including its salts; to act on plants, their environment or on seed.

The present invention also provides agricultural compositions comprising at least one acylhydrazide of formula I and at least one auxiliary customary for formulating crop protection agents and a process for the preparation of such compositions.

Further surprisingly it has been found that the acylhydrazides of formula I can be used effectively for treating or preventing infections, preferably parasitic and/or bacterial infections.

Accordingly the present invention also provides acylhydrazides of formula I for use as a medicament.

Further the present invention relates also to a method of treating or preventing infections, preferably parasitic and/or bacterial infections in a subject, which comprises administering to the subject a therapeutically effective amount of at least one acylhydrazide of formula I as defined, including the pharmaceutically acceptable salts thereof.

Furthermore the present invention relates to the use of an acylhydrazide of formula I as defined, including its pharmaceutically acceptable salts, for the preparation of a medicament for the therapeutic and/or prophylactic treatment of infections.

In addition the present invention provides pharmaceutical compositions comprising at least one acylhydrazide of formula I and at least one auxiliary customary for formulating pharmaceuticals and a process for the preparation of such pharmaceutical compositions.

The present invention also provides acylhydrazides of formula IA, which correspond to acylhydrazides of the formula I, wherein in case R² and R³ together with the carbon atoms (a) and (b) to which they are attached form a six-membered monocyclic cycle, said six-membered monocyclic cycle is partially or fully halogenated and/or is substituted by one to three substituents selected from the group as defined herein. Accordingly, since the acylhydrazides of formula IA represent a preferred embodiment of the acylhydrazides of formula I, hereinbelow the term "acylhydrazides of formula I" includes also the "acylhydrazides of formula IA".

Moreover, the invention relates to processes and intermediates for preparing acylhydrazides of formula I.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

As used herein, the terms "controlling" and "combating" are synonyms.

As used herein, the terms "undesirable vegetation" and "harmful plants" are synonyms.

The acylhydrazides of formula I as described herein are capable to form the following different tautomeric forms (Ia), (Ib) and (Ic):

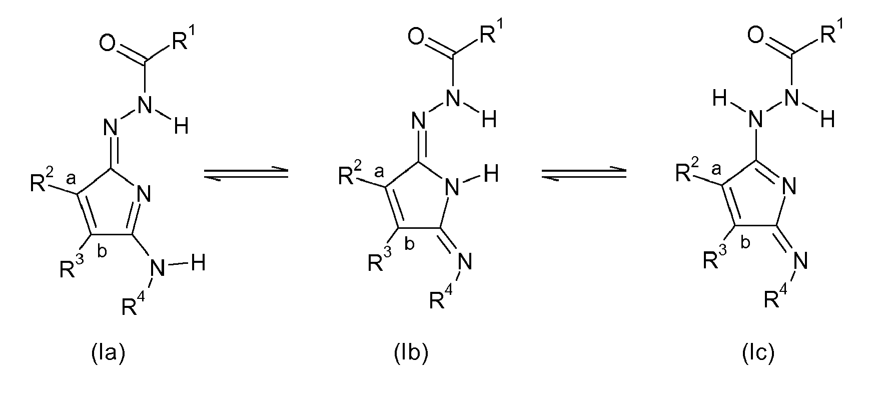

All possible tautomeric forms (Ia), (Ib) and (Ic) of the acylhydrazides of formula I or formula IA are contemplated to be within the scope of the present invention. Accordingly hereinbelow the term "acylhydrazides of formula I" or "acylhydrazides of formula IA" includes all tautomeric forms (Ia), (Ib) and (Ic).

In particular the acylhydrazides of formula I may exist in the tautomeric form (Ia).

If the acylhydrazides of formula I as described herein are capable of forming geometrical isomers, for example E/Z isomers, it is possible to use both, the pure isomers and mixtures thereof, in the compositions according to the invention.

If the acylhydrazides of formula I as described herein have one or more centers of chirality and, as a consequence, are present as enantiomers or diastereomers, it is possible to use both, the pure enantiomers and diastereomers and their mixtures, in the compositions according to the invention.

The organic moieties mentioned in the definition of the variables R¹ to R¹⁷, are - like the term halogen - collective terms for individual enumerations of the individual group members. The term halogen denotes in each case fluorine, chlorine, bromine or iodine. All hydrocarbon chains, i.e. all alkyl, can be straight-chain or branched, the prefix Cₙ-Cₘ denoting in each case the possible number of carbon atoms in the group.

Examples of such meanings are:
- C₁-C₄-alkyl: for example CH₃, C₂H₅, n-propyl, CH(CH₃)₂, n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-hydroxyalkyl, C₁-C₆-alkylthio, C₁-C₆-hydroxyalkylthio, C₁-C₆-hydroxyalkylamino, amino-C₁-C₆-alkylthio, (C₁-C₆-alkyl)amino-C₁-C₆-alkylthio, di(C₁-C₆-alkyl)amino-C₁-C₆-alkylthio, C₁-C₆-alkylamino, di(C₁-C₆-alkyl)amino, (C₁-C₆-alkyl)aminocarbonyl, di(C₁-C₆-alkyl)aminocarbonyl, C₁-C₆-alkylsulfinyl, C₁-C₆-alkylsulfonyl, C₁-C₆-hydroxyalkylsulfonyl, C₁-C₆-alkylsulfonyloxy, (C₁-C₆-alkyl)aminocarbonyl(C₁-C₆-alkyl)oxy, di(C₁-C₆-alkyl)aminocarbonyloxy, C₁-C₆-alkylsulfonylamino, N(C₁-C₆-alkylsulfonyl)-N(C₁-C₆-alkyl)amino, amino-C₁-C₆-alkylsulfonyl, (C₁-C₆-alkyl)amino-C₁-C₆-alkylsulfonyl, di(C₁-C₆-alkyl)amino-C₁-C₆-alkylsulfonyl, (C₁-C₆-alkyl)aminosulfonyl, di(C₁-C₆-alkyl)aminosulfonyl, C₁-C₆-alkylcarbonyl, C₁-C₆-alkylcarbonyloxy, amino-C₁-C₆-alkyl, (C₁-C₆-alkyl)amino-C₁-C₆-alkyl, di(C₁-C₆-alkyl)amino-C₁-C₆-alkyl, amino-C₁-C₆-alkylamino, (C₁-C₆-alkyl)amino-C₁-C₆-alkylamino, di(C₁-C₆-alkyl)amino-C₁-C₆-alkylamino, C₁-C₆-alkylsulfonylamino, C₁-C₆-alkylsulfonyl-(C₁-C₆-alkyl)amino, (C₁-C₆-alkyl)aminosulfonylamino, di(C₁-C₆-alkyl)aminosulfonylamino, formyl(C₁-C₆-alkyl)amino, (C₁-C₆-alkyl)carbonylamino, N(C₁-C₆-alkylcarbonyl)-N(C₁-C₆-alkyl)amino, (C₁-C₆-alkyl)aminocarbonylamino, di(C₁-C₆-alkyl)aminocarbonylamino, (C₁-C₆-alkyl)aminocarbonyl(C₁-C₆-alkyl)amino, N(phenyl)-N(C₁-C₆-alkyl)amino, N-phenylcarbonyl-N(C₁-C₆-alkyl)amino, C₁-C₆-alkoxy-C₁-C₆-alkyl, C₁-C₆-haloalkoxy-C₁-C₆-alkyl, (C₁-C₆-alkyl)amino-C₂-C₆-alkenyl, di(C₁-C₆-alkyl)amino-C₂-C₆-alkenyl, (C₁-C₆-alkyl)amino-C₂-C₆-alkynyl, di(C₁-C₆-alkyl)amino-C₂-C₆-alkynyl, (C₁-C₆-alkyl)amino-C₁-C₆-alkoxy, di(C₁-C₆-alkyl)amino-C₁-C₆-alkoxy, C₁-C₆-alkoxy-C₁-C₆-alkylthio, C₁-C₆-haloalkoxy-C₁-C₆-alkylthio, C₁-C₆-alkoxy-C₁-C₆-alkylamino, C₁-C₆-haloalkoxy-C₁-C₆-alkylamino, C₁-C₆-alkoxy-C₁-C₆-alkylsulfonyl, C₁-C₆-haloalkoxy-C₁-C₆-alkylsulfonyl, heterocydyl-C₁-C₆-alkyl, heterocydyl-C₁-C₆-alkylthio, heterocydyl-C₁-C₆-alkylthio, heterocyclyl-C₁-C₆-alkylamino, heterocydyl-C₁-C₆-alkylsulfonyl, heterocydyl-[(C₁-C₆-alkyl)-amino], heterocyclylcarbonyl[(C₁-C₆-alkyl)amino]: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₁-C₄-haloalkyl: a C₁-C₄-alkyl radical as mentioned above which is partially or fully substituted by fluorine, chlorine, bromine and/or iodine, for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyla C₁-C₃-haloalkyl radical as mentioned above, and also, for example, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;

- C₁-C₆-haloalkyl: C₁-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- C₃-C₆-cycloalkyl and also the cycloalkyl moieties of C₃-C₆-cycloalkylthio, C₃-C₆-cycloalkoxy, C₃-C₆-cycloalkylamino and C₃-C₆-cycloalkylsulfonyl: monocyclic saturated hydrocarbons having 3 to 6 ring members, such as cyclopropyl, cyclobutyl, cyclopentyl and cyclohexyl;
- C₃-C₆-alkenyl and also the C₃-C₆-alkenyl moieties of C₃-C₆-alkenyloxy, C₃-C₆-alkenylthio, C₃-C₆-alkenyamino, C₃-C₆-alkenylsulfonyl: for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- C₂-C₆-alkenyl and also the C₂-C₆-alkenyl moieties of C₂-C₆-hydroxyalkenyl, C₂-C₆-haloalkenyl, amino-C₂-C₆-alkenyl, C₁-C₆-alkoxy-C₂-C₆-alkenyl, C₁-C₆-haloalkoxy-C₂-C₆-alkenyl, (C₁-C₆-alkyl)amino-C₂-C₆-alkenyl, di(C₁-C₆-alkyl)amino-C₂-C₆-alkenyl, heterocyclyl-C₂-C₆-alkenyl: C₃-C₆-alkenyl as mentioned above, and also ethenyl;
- C₂-C₆-haloalkenyl: a C₃-C₆-alkenyl radical as mentioned above which is partially or fully substituted by fluorine, chlorine, bromine and/or iodine, for example 2-chlorovinyl, 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromovinyl, 2-bromoprop-2-en-1-yl, 3-bromoprop-2-en-1-yl, 2,3-dibromoprop-2-en-1-yl, 3,3-dibromoprop-2-en-1-yl, 2,3,3-tribromo-2-en-1-yl or 2,3-dibromobut-2-en-1-yl;
- C₃-C₆-alkynyl and also the C₃-C₆-alkenyl moieties of C₃-C₆-alkynyloxy, C₃-C₆-alkynylthio, C₃-C₆-alkynyamino, C₃-C₆-alkynylsulfonyl: for example 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
- C₂-C₆-alkynyl and also the C₂-C₆-alkynyl moieties of C₂-C₆-haloalkynyl, amino-C₂-C₆-alkynyl, C₁-C₆-hydroxyalkynyl, C₁-C₆-alkoxy-C₂-C₆-alkynyl, C₁-C₆-haloalkoxy-C₂-C₆-alkynyl, (C₁-C₆-alkyl)amino-C₂-C₆-alkynyl, di(C₁-C₆-alkyl)amino-C₂-C₆-alkynyl, heterocyclyl-C₂-C₆-alkynyl: C₃-C₆-alkynyl as mentioned above, and also ethynyl;
- C₃-C₆-haloalkynyl: a C₃-C₆-alkynyl radical as mentioned above which is partially or fully substituted by fluorine, chlorine, bromine and/or iodine, for example 1,1-difluoroprop-2-yn-1-yl, 3-chloroprop-2-yn-1-yl, 3-bromoprop-2-yn-1-yl, 3-iodoprop-2-yn-1-yl, 4-fluorobut-2-yn-1-yl, 4-chlorobut-2-yn-1-yl, 1,1-difluorobut-2-yn-1-yl, 4-iodobut-3-yn-1-yl, 5-fluoropent-3-yn-1-yl, 5-iodopent-4-yn-1-yl, 6-fluorohex-4-yn-1-yl or 6-iodohex-5-yn-1-yl;
- C₁-C₄-alkoxy: for example methoxy, ethoxy, propoxy, 1-methylethoxy butoxy, 1-methylpropoxy, 2-methylpropoxy and 1,1-dimethylethoxy;
- C₁-C₆-alkoxy and also the C₁-C₆-alkoxy moieties of C₁-C₆-hydroxyalkoxy, C₁-C₆-alkoxycarbonyl, C₁-C₆-alkoxy-C₁-C₆-alkoxy, amino-C₁-C₆-alkoxy, C₁-C₆-alkoxy-C₁-C₆-alkyl, C₁-C₆-alkoxy-C₂-C₆-alkenyl, C₁-C₆-alkoxy-C₂-C₆-alkynyl, C₁-C₆-haloalkoxy-C₁-C₆-alkoxy, (C₁-C₆-alkyl)amino-C₁-C₆-alkoxy, di(C₁-C₆-alkyl)amino-C₁-C₆-alkoxy, C₁-C₆-alkoxy-C₁-C₆-alkylthio, C₁-C₆-alkoxy-C₁-C₆-alkylamino, C₁-C₆-alkoxy-C₁-C₆-alkylsulfonyl, heterocyclyl-C₁-C₆-alkoxy: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy;
- C₁-C₄-haloalkoxy: a C₁-C₄-alkoxy radical as mentioned above which is partially or fully substituted by fluorine, chlorine, bromine and/or iodine, i.e., for example, fluoromethoxy, difluoromethoxy, trifluoromethoxy, chlorodifluoromethoxy, bromodifluoromethoxy, 2-fluoroethoxy, 2-chloroethoxy, 2-bromomethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy, 2-fluoropropoxy, 3-fluoropropoxy, 2-chloropropoxy, 3-chloropropoxy, 2-bromopropoxy, 3-bromopropoxy, 2,2-difluoropropoxy, 2,3-difluoropropoxy, 2,3-dichloropropoxy, 3,3,3-trifluoropropoxy, 3,3,3-trichloropropoxy, 2,2,3,3,3-pentafluoropropoxy, heptafluoropropoxy, 1-(fluoromethyl)-2-fluoroethoxy, 1-(chloromethyl)-2-chloroethoxy, 1-(bromomethyl)-2-bromoethoxy, 4-fluorobutoxy, 4-chlorobutoxy, 4-bromobutoxy and nonafluorobutoxy;
- C₁-C₆-haloalkoxy and the C₁-C₆-haloalkoxy moieties of C₁-C₆-haloalkoxy-C₁-C₆-alkyl, C₁-C₆-haloalkoxy-C₂-C₆-alkenyl, C₁-C₆-haloalkoxy-C₂-C₆-alkynyl, C₁-C₆-haloalkoxy-C₁-C₆-alkoxy, C₁-C₆-haloalkoxy-C₁-C₆-alkylthio, C₁-C₆-haloalkoxy-C₁-C₆-alkylamino, C₁-C₆-haloalkoxy-C₁-C₆-alkylsulfonyl: C₁-C₄-haloalkoxy as mentioned above, and also, for example, 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy and dodecafluorohexoxy;
- heterocyclyl and the heterocyclyl moieties of heterocyclyl-C₁-C₆-alkyl, heterocyclyl-C₂-C₆-alkenyl, heterocyclyl-C₂-C₆-alkynyl, heterocyclyl-C₁-C₆-alkoxy, heterocyclyl-C₁-C₆-alkylthio, heterocydyl-C₁-C₆-alkylamino, heterocydyl-C₁-C₆-alkylsulfonyl, heterocyclyloxy, heterocyclylthio, heterocyclylsulfinyl, heterocyclylsulfonyl, heterocyclylsulfonyloxy, heterocyclylcarbonyl, heterocyclylcarbonyloxy, heterocyclylamino, heterocyclyl-[(C₁-C₆-alkyl)amino], heterocyclylcarbonylamino, heterocydylcarbonyl[(C₁-C₆-alkyl)-amino]: saturated, partially unsaturated or aromatic 3- to 6-membered heterocycle which, in addition to carbon atoms, comprises one to three nitrogen atoms, one or two nitrogen atoms and one sulfur atom, one nitrogen atom and one oxygen atom, one oxygen atoms, one or two sulfur atoms, one oxygen atom, or one sulfur atom, for example 3- and 4-membered rings like 2-oxiranyl, 2-oxetanyl, 3-oxetanyl, 2-aziridinyl, 2-thiiranyl, 2-thiethanyl, 3-thiethanyl, 1-azetidinyl, 2-azetidinyl, 1-azetinyl, 2-azetinyl; 5-membered saturated rings like 2-tetrahydrofuranyl, 3-tetrahydrofuranyl, 2-tetrahydrothienyl, 3-tetrahydrothienyl, 1-pyrrolidinyl, 2-pyrrolidinyl, 3-pyrrolidinyl, 3-isoxazolidinyl, 4-isoxazolidinyl, 5-isoxazolidinyl, 2-isothiazolidinyl, 3-isothiazolidinyl, 4-isothiazolidinyl, 5-isothiazolidinyl, 1-pyrazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 1-imidazolidinyl, 2-imidazolidinyl, 4-imidazolidinyl, 3-oxazolidinyl, 1,2,4-oxadiazolidin-3-yl, 1,2,4-oxadiazolidin-5-yl, 3-thiazolidinyl, 1,2,4-thiadiazolidin-3-yl, 1,2,4-thiadiazolidin-5-yl, 1,2,4-triazolidin-3-yl, 1,2,4-oxadiazolidin-2-yl, 1,2,4-oxadiazolidin-4-yl, 1,3,4-oxadiazolidin-2-yl, 1,2,4-thiadiazolidin-2-yl, 1,2,4-thiadiazolidin-4-yl, 1,3,4-thiadiazolidin-2-yl, 1,2,4-triazolidin-1-yl, 1,3,4-triazolidin-2-yl; 5-membered partially unsaturated rings like 2,3-dihydrofur-2-yl, 2,3-dihydrofur-3-yl, 2,4-dihydrofur-2-yl, 2,4-dihydrofur-3-yl, dioxolan-2-yl, 1,3-dioxol-2-yl, 2,3-dihydrothien-2-yl, 2,3-dihydrothien-3-yl, 2,4-dihydrothien-2-yl, 2,4-dihydrothien-3-yl, 4,5-dihydropyrrol-1-yl, 4,5-dihydropyrrol-2-yl, 4,5-dihydropyrrol-3-yl, 2,5-dihydropyrrol-1-yl, 2,5-dihydropyrrol-2-yl, 2,5-dihydropyrrol-3-yl, 2,3-dihydroisoxazol-1-yl, 2,3-dihydroisoxazol-3-yl, 2,3-dihydroisoxazol-4-yl, 2,3-dihydroisoxazol-5-yl, 2,5-dihydroisoxazol-3-yl, 2,5-dihydroisoxazol-4-yl, 2,5-dihydroisoxazol-5-yl, 4,5-dihydroisoxazol-2-yl, 4,5-dihydroisoxazol-3-yl, 4,5-dihydroisoxazol-4-yl, 4,5-dihydroisoxazol-5-yl, 2,3-dihydroisothiazol-1-yl, 2,3-dihydroisothiazol-3-yl, 2,3-dihydroisothiazol-4-yl, 2,3-dihydroisothiazol-5-yl, 2,5-dihydroisothiazol-3-yl, 2,5-dihydroisothiazol-4-yl, 2,5-dihydroisothiazol-5-yl, 4,5-dihydroisothiazol-1-yl, 4,5-dihydroisothiazol-3-yl, 4,5-dihydroisothiazol-4-yl, 4,5-dihydroisothiazol-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydroimidazol-1-yl, 2,3-dihydroimidazol-2-yl, 2,3-dihydroimidazol-3-yl ,2,3-dihydroimidazol-4-yl, 2,3-dihydroimidazol-5-yl, 4,5-dihydroimidazol-1-yl, 4,5-dihydroimidazol-2-yl, 4,5-dihydroimidazol-4-yl, 4,5-dihydroimidazol-5-yl, 2,5-dihydroimidazol-1-yl, 2,5-dihydroimidazol-2-yl, 2,5-dihydroimidazol-4-yl, 2,5-dihydroimidazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 2,3-dihydrothiazol-2-yl, 2,3-dihydrothiazol-3-yl, 2,3-dihydrothiazol-4-yl, 2,3-dihydrothiazol-5-yl, 3,4-dihydrothiazol-2-yl, 3,4-dihydrothiazol-3-yl, 3,4-dihydrothiazol-4-yl, 3,4-dihydrothiazol-5-yl, 3,4-dihydrothiazol-2-yl, 3,4-dihydrothiazol-3-yl, 3,4-dihydrothiazol-4-yl; 5-membered aromatic rings like furyl (for example 2-furyl, 3-furyl), thienyl (for example 2-thienyl, 3-thienyl), pyrrolyl (for example pyrrol-2-yl, pyrrol-3-yl), pyrazolyl (for example pyrazol-3-yl, pyrazol-4-yl), isoxazolyl (for example isoxazol-3-yl, isoxazol-4-yl, isoxazol-5-yl), isothiazolyl (for example isothiazol-3-yl, isothiazol-4-yl, isothiazol-5-yl), imidazolyl (for example imidazol-2-yl, imidazol-4-yl), oxazolyl (for example oxazol-2-yl, oxazol-4-yl, oxazol-5-yl), thiazolyl (for example thiazol-2-yl, thiazol-4-yl, thiazol-5-yl), oxadiazolyl (for example 1,2,3-oxadiazol-4-yl, 1,2,3-oxadiazol-5-yl, 1,2,4-oxadiazol-3-yl, 1,2,4,-oxadiazol-5-yl, 1,3,4-oxadiazol-2-yl), thiadiazolyl (for example 1,2,3-thiadiazol-4-yl, 1,2,3-thiadiazol-5-yl, 1,2,4-thiadiazol-3-yl, 1,2,4-thiadiazol-5-yl, 1,3,4-thiadiazolyl-2-yl), triazolyl (for example 1,2,3-triazol-4-yl, 1,2,4-triazol-3-yl); 6-membered saturated rings like 1-piperidinyl, 2-piperidinyl, 3-piperidinyl, 4-piperidinyl, 1,3-dioxan-5-yl, 1,4-dioxanyl, 1,3-dithian-5-yl, 1,3-dithianyl, 1,3-oxathian-5-yl, 1,4-oxathianyl, 2-tetrahydropyranyl, 3-tetrahydopyranyl, 4-tetrahydropyranyl, 2-tetrahydrothiopyranyl, 3-tetrahydrothiopyranyl,4-tetrahydrothiopyranyl, 1-hexahydropyridazinyl, 3-hexahydropyridazinyl, 4-hexahydropyridazinyl, 1-hexahydropyrimidinyl, 2-hexahydropyrimidinyl, 4-hexahydropyrimidinyl, 5-hexahydropyrimidinyl, 1-piperazinyl, 2-piperazinyl, 1,3,5-hexahydrotriazin-1-yl, 1,3,5-hexahydrotriazin-2-yl, 1,2,4-hexahydrotriazin-1-yl, 1,2,4-hexahydrotriazin-3-yl, tetrahydro-1,3-oxazin-1-yl, tetrahydro-1,3-oxazin-2-yl, tetrahydro-1,3-oxazin-6-yl, 1-morpholinyl, 2-morpholinyl, 3-morpholinyl; 6-membered partially unsaturated rings like 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 2H-thiopyran-6-yl, 5,6-dihydro-4H-1,3-oxazin-2-yl; 6-membered aromatic rings like pyridyl (for example pyridin-2-yl, pyridin-3-yl, pyridin-4-yl), pyrazinyl (for example pyridazin-3-yl, pyridazin-4-yl), pyrimidinyl (for example pyrimidin-2-yl, pyrimidin-4-yl, pyrimidin-5-yl), pyrazin-2-yl, triazinyl (for example 1,3,5-triazin-2-yl, 1,2,4-triazin-3-yl, 1,2,4-triazin-5-yl, 1,2,4-triazin-6-yl); and also bicycles such as the benzo-fused derivatives of the abovementioned monocycles, for example quinolinyl, isoquinolinyl, indolyl, benzothienyl, benzofuranyl, benzoxazolyl, benzothiazolyl, benzisothiazolyl, benzimidazolyl, benzopyrazolyl, benzothiadiazolyl, benzotriazolyl;
- 5- or 6-membered monocyclic or eight- to ten-membered bicyclic heteroaryl:
   aromatic 5- to 10-membered mono- or bicyclic heterocycle which, in addition to carbon atoms comprises one to three nitrogen atoms, one or two nitrogen atoms and one sulfur atom, one nitrogen and one oxygen atom, one oxygen atom, or one sulfur atom as ring members, for example monocycles, such as 5-membered aromatic rings as mentioned above; 6-membered aromatic rings as mentioned above; and also bicycles such as the benzo-fused derivatives of the above-mentioned monocycles, for example quinolinyl, isoquinolinyl, indolyl, benzothienyl, benzofuranyl, benzoxazolyl, benzothiazolyl, benzisothiazolyl, benzimidazolyl, benzopyrazolyl, benzothiadiazolyl, benzotriazolyl;
- five- or six-membered heterocyclyl: saturated, partially unsaturated or aromatic 5-or 6-membered heterocycle which, in addition to carbon atoms, comprises one or two nitrogen atoms, one nitrogen and one oxygen atom or one nitrogen and one sulfur atom, for example 5-membered saturated rings as mentioned above, 5-membered partially unsaturated rings as mentioned above, 5-membered aromatic rings as mentioned above, 6-membered saturated rings as mentioned above, 6-membered partially unsaturated rings as mentioned above, 6-membered aromatic rings as mentioned above;
- five- or six-membered monocyclic or eight- to ten-membered bicyclic cyclus: partially unsaturated or aromatic 5- to 10-membered mono- or bicyclic heterocycle, which, in addition to carbon atoms, may comprise one to three nitrogen atoms, one or two nitrogen atoms and one sulfur atom, one nitrogen and one oxygen atom, one or two sulfur atoms, or one or two oxygen atoms, for example monocycles like 5-membered saturated rings as mentioned above and cyclopentyl, 5-membered partially unsaturated rings as mentioned above and *cyclopentenyl, 5-membered aromatic rings as mentioned above, 6-membered saturated rings as mentioned above and cyclohexyl, 6-membered partially unsaturated rings as mentioned above and cyclohexenyl, 6-membered aromatic rings as mentioned above and phenyl, and also bicycles such as the benzo-fused derivatives of the abovementioned monocycles, for example naphtyl, anthracenyl, quinolinyl, isoquinolinyl, indolyl, benzothienyl, benzofuranyl, benzoxazolyl, benzothiazolyl, benzisothiazolyl, benzimidazolyl, benzopyrazolyl, benzothiadiazolyl, benzotriazolyl.

The preferred embodiments of the invention mentioned herein below have to be understood as being preferred either independently from each other or in combination with one another.

According to a preferred embodiment of the invention preference is also given to those acylhydrazides of formula I wherein the variables, either independently of one another or in combination with one another, have the following meanings:
R¹ preferably is a five- or six-membered monocyclic or eight- to ten-membered bicyclic heteroaryl having one to three nitrogen atoms, or one nitrogen and one oxygen atom, which may be partially or fully halogenated and/or may be substituted by
   one substituent selected from the group consisting of C₁-C₆-alkyl, C₃-C₆-cycloalkyl, C₁-C₆-haloalkyl, C₂-C₆-alkenyl, C₂-C₆-alkynyl, C₁-C₆-alkoxy, C₃-C₆-cycloalkoxy, C₁-C₆-haloalkoxy, C₃-C₆-alkenyloxy, C₃-C₆-alkynyloxy, C₁-C₆-alkylthio, C₁-C₆-alkylsulfonyl, amino, C₁-C₆-alkylamino, di(C₁-C₆)alkylamino, C₁-C₆-alkylsulfonylamino, N(C₁-C₆-alkylsulfonyl)-N(C₁-C₆-alkyl)amino, five- or six-membered heterocyclyl having one or two nitrogen atoms, one nitrogen and one oxygen atom or one nitrogen and one sulfur atom, or phenyl, which itself may be partially or fully halogenated
   and/or may be substituted one to three substituents selected from of the group consisting of C₁-C₆-alkyl, C₁-C₆-haloalkyl and C₁-C₆-alkoxy;
   more preferred is a six-membered monocyclic heteroaryl having one to three nitrogen atoms, which which may be partially or fully halogenated and/or may be substituted by one substituent selected from the group consisting of C₁-C₆-alkyl, C₃-C₆-cycloalkyl, C₁-C₆-haloalkyl, C₂-C₆-alkenyl, C₂-C₆-alkynyl, C₁-C₆-alkoxy, C₃-C₆-cycloalkoxy, C₁-C₆-haloalkoxy, C₃-C₆-alkenyloxy, C₃-C₆-alkynyloxy, di(C₁-C₆)alkylamino, five- or six-membered heterocyclyl having one or two nitrogen atoms or one nitrogen and one oxygen atom, or phenyl, which itself may be partially or fully halogenated and/or may be substituted one to three substituents selected from of the group consisting of C₁-C₆-alkyl, C₁-C₆-haloalkyl and C₁-C₆-alkoxy;
   particularly preferred is pyridyl;
   especially preferred is 2-pyridyl or 4-pyridyl;
   most preferred is 2-pyridyl;
   wherein each of the four pyridyl moieties mentioned before preferably may be partially or fully halogenated and/or may be substituted by one substituent selected from the group consisting of C₁-C₆-alkyl, C₃-C₆-cycloalkyl, C₁-C₆-haloalkyl, C₂-C₆-alkenyl, C₂-C₆-alkynyl, C₁-C₆-alkoxy, C₃-C₆-cycloalkoxy, C₁-C₆-haloalkoxy, C₃-C₆-alkenyloxy, C₃-C₆-alkynyloxy, di(C₁-C₆)alkylamino, five- or six-membered heterocyclyl having one or two nitrogen atoms or one nitrogen and one oxygen atom, or phenyl, which itself may be partially or fully halogenated and/or may be substituted one to three substituents selected from of the group consisting of C₁-C₆-alkyl, C₁-C₆-haloalkyl and C₁-C₆-alkoxy;
   more preferred is partially halogenated and/or is substituted by one substituent selected from the group consisting of C₁-C₆-alkyl, C₁-C₆-haloalkyl, C₁-C₆-alkoxy, di(C₁-C₆)alkylamino or phenyl, which itself may be partially or fully halogenated and/or may be substituted one to three substituents selected from of the group consisting of C₁-C₆-alkyl, C₁-C₆-haloalkyl and C₁-C₆-alkoxy;
   also particularly preferred is selected from the group consisting of A1, A2 and A3: 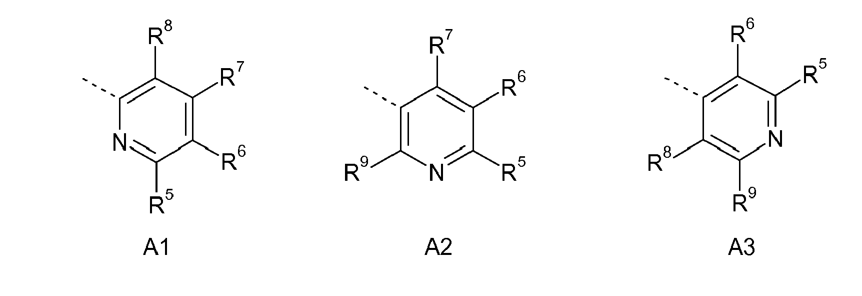 wherein R⁵, R⁶, R⁷, R⁸ and R⁹ are as defined below:
   R⁵ hydrogen;
   R⁶ hydrogen, halogen or C₁-C₆-alkyl, preferably hydrogen;
   R⁷ hydrogen, halogen, C₁-C₆-alkyl, C₁-C₆-alkoxy, di(C₁-C₆)alkylamino or phenyl; preferably hydrogen, C₁-C₆-alkyl, C₁-C₆-alkoxy or di(C₁-C₆)alkylamino;
   R⁸ hydrogen or C₁-C₆-alkyl;
   R⁹ hydrogen;
   R² and R³ preferably together with the carbon atoms (a) and (b) to which they are attached form a five- or six-membered monocyclic or eight- to ten-membered bicyclic cyclus, which is partially unsaturated or aromatic, which may contain one to three nitrogen atoms, one or two nitrogen atoms and one sulfur atom, one nitrogen and one oxygen atom, one or two sulfur atoms, or one or two oxygen atoms; and
   which may be partially or fully halogenated and/or may be substituted by one to three substituents selected from the group consisting of C₁-C₆-alkyl, C₃-C₆-cycloalkyl, C₁-C₆-haloalkyl, C₁-C₆-alkoxy-C₁-C₆-alkyl, C₁-C₆-haloalkoxy-C₁-C₆-alkyl, (C₁-C₆-alkyl)amino-C₁-C₆-alkyl, di(C₁-C₆-alkyl)amino-C₁-C₆-alkyl, C₂-C₆-alkenyl, C₂-C₆-haloalkenyl, C₁-C₆-alkoxy-C₂-C₆-alkenyl, C₁-C₆-haloalkoxy-C₂-C₆-alkenyl, (C₁-C₆-alkyl)-amino-C₂-C₆-alkenyl, di(C₁-C₆-alkyl)amino-C₂-C₆-alkenyl, C₂-C₆-alkynyl, C₂-C₆-haloalkynyl, C₁-C₆-alkoxy-C₂-C₆-alkynyl, C₁-C₆-haloalkoxy-C₂-C₆-alkynyl, (C₁-C₆-alkyl)amino-C₂-C₆-alkynyl, di(C₁-C₆-alkyl)amino-C₂-C₆-alkynyl, C₁-C₆-alkylcarbonyl, hydroxycarbonyl, C₁-C₆-alkoxycarbonyl, C₁-C₆-alkoxyC₃-C₆-cycloalkoxy, C₃-C₆-alkenyloxy, C₃-C₆-alkynyloxy, C₁-C₆-alkylcarbonyloxy, C₁-C₆-alkylsulfonyloxy, C₁-C₆-alkylthio, C₁-C₆-alkylsulfonylamino, C₁-C₆-alkylsulfinyl, C₁-C₆-alkylsulfonyl, aminosulfonyl, (C₁-C₆-alkyl)aminosulfonyl, di(C₁-C₆-alkyl)-aminosulfonyl, phenyl, phenyloxy, phenylthio, phenylsulfonyl, phenylsulfonyloxy, phenylamino, N(phenyl)-N(C₁-C₆-alkyl)amino,
   wherein each of the phenyl groups may be partially halogenated and/or may carry one substituents selected from the group consisting of C₁-C₆-alkyl, C₁-C₆-haloalkyl, C₁-C₆-alkoxy;
   heterocyclyl, heterocyclyl-C₁-C₆-alkyl, heterocyclyl-C₂-C₆-alkenyl, heterocyclyl-C₂-C₆-alkynyl, heterocyclyloxy, heterocyclylthio, heterocyclyl sulfinyl, each of the heterocyclyl groups being three- to seven-membered and having one to three nitrogen atoms, one nitrogen atom and one sulfur atom, or one nitrogen atom and one oxygen atom,
   wherein each of the heterocyclyl groups may be partially halogenated and/or may be substituted with one to three substituents selected from the group consisting of C₁-C₆-alkyl, C₁-C₆-haloalkyl, hydroxy, C₁-C₆-alkoxy, C₁-C₆-alkylcarbonyloxy;
   more preferred together with the carbon atoms (a) and (b) to which they are attached form a five- or six-membered monocyclic or eight- to ten-membered bicyclic cyclus, which is partially unsaturated or aromatic, which may contain one to three nitrogen atoms, one or two nitrogen atoms and one sulfur atom, one nitrogen and one oxygen atom, one or two sulfur atoms, or one or two oxygen atoms; and
   which may be partially or fully halogenated and/or may be substituted by one to three substituents selected from the group consisting of C₁-C₆-alkyl, C₃-C₆-cycloalkyl, C₁-C₆-haloalkyl, C₁-C₆-alkoxy-C₁-C₆-alkyl, C₁-C₆-haloalkoxy-C₁-C₆-alkyl, (C₁-C₆-alkyl)amino-C₁-C₆-alkyl, di(C₁-C₆-alkyl)amino-C₁-C₆-alkyl, C₂-C₆-alkenyl, C₂-C₆-haloalkenyl, C₁-C₆-alkoxy-C₂-C₆-alkenyl, C₁-C₆-haloalkoxy-C₂-C₆-alkenyl, (C₁-C₆-alkyl)-amino-C₂-C₆-alkenyl, di(C₁-C₆-alkyl)amino-C₂-C₆-alkenyl, C₂-C₆-alkynyl, C₂-C₆-haloalkynyl, C₁-C₆-alkoxy-C₂-C₆-alkynyl, C₁-C₆-haloalkoxy-C₂-C₆-alkynyl, (C₁-C₆-alkyl)amino-C₂-C₆-alkynyl, di(C₁-C₆-alkyl)amino-C₂-C₆-alkynyl, C₁-C₆-alkylcarbonyl, C₁-C₆-alkoxyC₃-C₆-cycloalkoxy, C₃-C₆-alkenyloxy, C₃-C₆-alkynyloxy, C₁-C₆-alkylcarbonyloxy, C₁-C₆-alkylthio, C₁-C₆-alkylsulfonyl, aminosulfonyl, (C₁-C₆-alkyl)aminosulfonyl, di(C₁-C₆-alkyl)aminosulfonyl, phenyl, phenyloxy, phenylthio,
   wherein each of the phenyl groups may be partially halogenated and/or may carry one substituents selected from the group consisting of C₁-C₆-alkyl, C₁-C₆-haloalkyl, C₁-C₆-alkoxy; heterocyclyl-C₁-C₆-alkyl, heterocyclyl-C₂-C₆-alkenyl, heterocyclyl-C₂-C₆-alkynyl, heterocyclyloxy, heterocyclylthio, each of the heterocyclyl groups being three- to seven-membered and having one to three nitrogen atoms, one nitrogen atom and one sulfur atom, or one nitrogen atom and one oxygen atom, wherein each of the heterocyclyl groups may be partially halogenated and/or may be substituted with one to three substituents selected from the group consisting of C₁-C₆-alkyl, C₁-C₆-haloalkyl, hydroxy, C₁-C₆-alkoxy, C₁-C₆-alkylcarbonyloxy; particularly preferred together with the carbon atoms (a) and (b) to which they are attached form six-membered monocyclic aromatic or ten-membered bicyclic aromatic cyclus,
   which may be partially or fully halogenated and/or may be substituted by one to three substituents selected from the group consisting of C₁-C₆-alkyl, C₃-C₆-cycloalkyl, C₁-C₆-haloalkyl, C₁-C₆-alkoxy-C₁-C₆-alkyl, C₁-C₆-haloalkoxy-C₁-C₆-alkyl, (C₁-C₆-alkyl)amino-C₁-C₆-alkyl, di(C₁-C₆-alkyl)amino-C₁-C₆-alkyl, C₂-C₆-alkenyl, C₂-C₆-haloalkenyl, C₁-C₆-alkoxy-C₂-C₆-alkenyl, C₁-C₆-haloalkoxy-C₂-C₆-alkenyl, (C₁-C₆-alkyl)-amino-C₂-C₆-alkenyl, di(C₁-C₆-alkyl)amino-C₂-C₆-alkenyl, C₂-C₆-alkynyl, C₂-C₆-haloalkynyl, C₁-C₆-alkoxy-C₂-C₆-alkynyl, C₁-C₆-haloalkoxy-C₂-C₆-alkynyl, (C₁-C₆-alkyl)amino-C₂-C₆-alkynyl, di(C₁-C₆-alkyl)amino-C₂-C₆-alkynyl, C₁-C₆-alkylcarbonyl, C₁-C₆-alkoxyC₃-C₆-cydoalkoxy, C₃-C₆-alkenyloxy, C₃-C₆-alkynyloxy, C₁-C₆-alkylcarbonyloxy, C₁-C₆-alkylthio, C₁-C₆-alkylsulfonyl, aminosulfonyl, (C₁-C₆-alkyl)aminosulfonyl, di(C₁-C₆-alkyl)aminosulfonyl, phenyl, phenyloxy, phenylthio,
   wherein each of the phenyl groups may be partially halogenated and/or may carry one substituents selected from the group consisting of C₁-C₆-alkyl, C₁-C₆-haloalkyl, C₁-C₆-alkoxy; heterocydyl-C₁-C₆-alkyl, heterocyclyl-C₂-C₆-alkenyl, heterocyclyl-C₂-C₆-alkynyl, heterocyclyloxy, heterocyclylthio, each of the heterocyclyl groups being three- to seven-membered and having one to three nitrogen atoms, one nitrogen atom and one sulfur atom, or one nitrogen atom and one oxygen atom, wherein each of the heterocyclyl groups may be partially halogenated and/or may be substituted with one to three substituents selected from the group consisting of C₁-C₆-alkyl, C₁-C₆-haloalkyl, hydroxy, C₁-C₆-alkoxy, C₁-C₆-alkylcarbonyloxy
   especially preferred together with the carbon atoms (a) and (b) to which they are attached form a cycle selected from the group consisting of B1, B2, B3 and B4 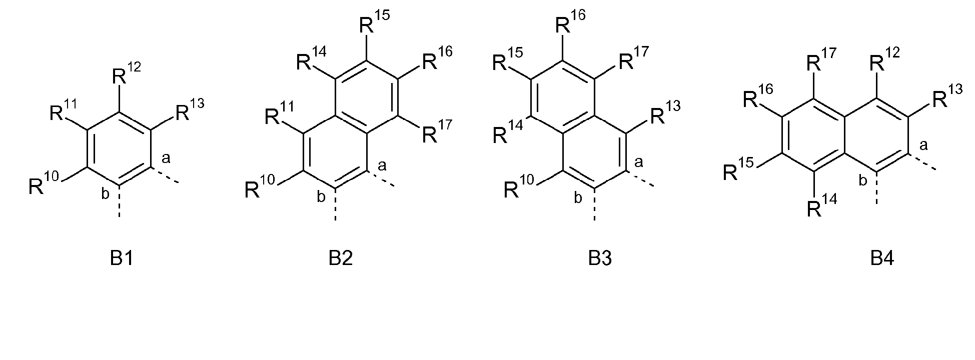 wherein
   R¹⁰ is hydrogen, halogen, nitro, C₁-C₆-alkyl, C₁-C₆-alkoxy, C₁-C₆-haloalkoxy, C₁-C₆-alkylthio, di(C₁-C₆-alkyl)amino-C₁-C₆-alkylthio, di(C₁-C₆-alkyl)aminosulfonyl, phenyl, phenyloxy or phenylthio, wherein each of the phenyl groups may be partially or fully halogenated, preferably may be substituted by one halogen atom, more preferably is substituted by one halogen atom;
   R¹¹ is hydrogen or halogen, preferably hydrogen or fluorine, more preferably hydrogen; is also preferably halogen, more preferably fluorine;
   R¹² is hydrogen or halogen, preferably hydrogen or fluorine, more preferably hydrogen; is also preferably halogen, more preferably fluorine;
   R¹³ is hydrogen or halogen, preferably hydrogen or fluorine, more preferably hydrogen, is also preferably halogen; more preferably fluorine;
   R¹⁴ is hydrogen or halogen, preferably hydrogen or fluorine, more preferably hydrogen; is also preferably halogen, more preferably fluorine
   R¹⁵ is hydrogen or halogen, preferably hydrogen or fluorine, more preferably hydrogen; is also preferably halogen, more preferably fluorine
   R¹⁶ is hydrogen or halogen, preferably hydrogen or fluorine, more preferably hydrogen; is also preferably halogen, more preferably fluorine
   R¹⁷ is hydrogen or halogen, preferably hydrogen or fluorine, more preferably hydrogen; is also preferably halogen, more preferably fluorine
   R⁴ preferably is C₁-C₆-alkylcarbonyl, also preferably is hydrogen.

According to another preferred embodiment of the invention preference is given to the acylhydrazides of formula IA, which correspond to acylhydrazides of the formula I, wherein in case R² and R³ together with the carbon atoms (a) and (b) to which they are attached form a six-membered monocyclic cycle, preferably the cycle B1, said six-membered monocyclic cycle is partially or fully halogenated and/or is substituted by one to three substituents selected from the group as defined herein.

With regard to the preferred embodiments of the acylhydrazides of formula IA the preferred embodiments mentioned herein with regard to the acylhydrazides of formula I are fully applicable.

According to another preferred embodiment of the invention preference is given to the acylhydrazides of formula IB, which correspond to acylhydrazides of the formula I, wherein in case R¹ is pyridyl, preferably a pyridyl selected from the group consisting A1, A2 and A3, more preferably the pyridy A1, said pyridyl is substituted by at least one substituent selected from the group as defined herein. "Substituted" in this context means that the pyridyl carries at least one substituent, which is not hydrogen.

With regard to the preferred embodiments of the acylhydrazides of formula IB the preferred embodiments mentioned herein with regard to the acylhydrazides of formula I are fully applicable.

Particular preference is given to acylhydrazides of the formula I.1 (which corresponds to formula I wherein R¹ is A1, wherein R⁵ = H and R⁸ = H; R² and R³ together form B1, wherein R¹¹ = H, R¹² = H and R¹³ = H; and R⁴ = H) 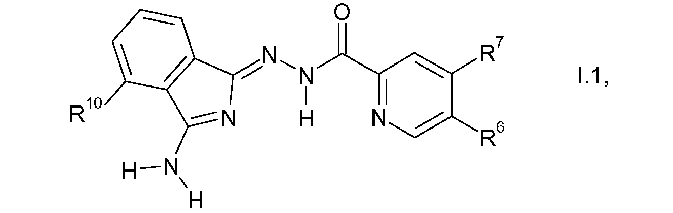 wherein the variables R⁶, R⁷, and R¹⁰ have the meanings, in particular the preferred meanings, as defined above.

Most preference to the compounds of the formulae I.1.1 to I.1.378 of Table 1, where the definitions of the variables R⁶, R⁷, and R¹⁰ are of particular importance for the compounds according to the invention not only in combination with one another but in each case also on their own:

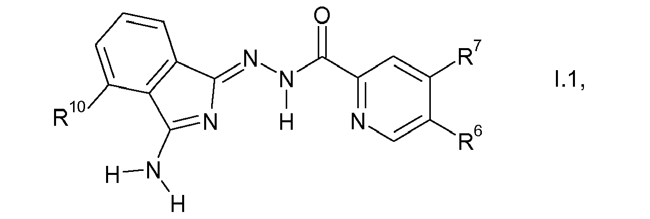

**Table 1**

| No. | R⁶ | R⁷ | R¹⁰ |
|---|---|---|---|
| I.1.1. | H | H | H |
| I.1.2. | H | H | F |
| I.1.3. | H | H | Cl |
| I.1.4. | H | H | NO₂ |
| I.1.5. | H | H | CH₃ |
| I.1.6. | H | H | OCH₃ |
| I.1.7. | H | H | OCH₂CF₃ |
| I.1.8. | H | H | SCH₃ |
| I.1.9. | H | H | SCH₂CH₂N(CH₃)₂ |
| I.1.10. | H | H | SO₂N(CH₃)₂ |
| I.1.11. | H | H | S-C₆H₅ |
| I.1.12. | H | H | O-C₆H₅ |
| I.1.13. | H | H | O-(4-F-C₆H₄) |
| I.1.14. | H | H | O-(4-Cl-C₆H₄) |
| I.1.15. | H | F | H |
| I.1.16. | H | F | F |
| I.1.17. | H | F | Cl |
| I.1.18. | H | F | NO₂ |
| I.1.19. | H | F | CH₃ |
| I.1.20. | H | F | OCH₃ |
| I.1.21. | H | F | OCH₂CF₃ |
| I.1.22. | H | F | SCH₃ |
| I.1.23. | H | F | SCH₂CH₂N(CH₃)₂ |
| I.1.24. | H | F | SO₂N(CH₃)₂ |
| I.1.25. | H | F | S-C₆H₅ |
| I.1.26. | H | F | O-C₆H₅ |
| I.1.27. | H | F | O-(4-F-C₆H₄) |
| I.1.28. | H | F | O-(4-Cl-C₆H₄) |
| I.1.29. | H | Cl | H |
| I.1.30. | H | Cl | F |
| I.1.31. | H | Cl | Cl |
| I.1.32. | H | Cl | NO₂ |
| I.1.33. | H | Cl | CH₃ |
| I.1.34. | H | Cl | OCH₃ |
| I.1.35. | H | Cl | OCH₂CF₃ |
| I.1.36. | H | Cl | SCH₃ |
| I.1.37. | H | Cl | SCH₂CH₂N(CH₃)₂ |
| I.1.38. | H | Cl | SO₂N(CH₃)₂ |
| I.1.39. | H | Cl | S-C₆H₅ |
| I.1.40. | H | Cl | O-C₆H₅ |
| I.1.41. | H | Cl | O-(4-F-C₆H₄) |
| I.1.42. | H | Cl | O-(4-Cl-C₆H₄) |
| I.1.43. | H | Br | H |
| I.1.44. | H | Br | F |
| I.1.45. | H | Br | Cl |
| I.1.46. | H | Br | NO₂ |
| I.1.47. | H | Br | CH₃ |
| I.1.48. | H | Br | OCH₃ |
| I.1.49. | H | Br | OCH₂CF₃ |
| I.1.50. | H | Br | SCH₃ |
| I.1.51. | H | Br | SCH₂CH₂N(CH₃)₂ |
| I.1.52. | H | Br | SO₂N(CH₃)₂ |
| I.1.53. | H | Br | S-C₆H₅ |
| I.1.54. | H | Br | O-C₆H₅ |
| I.1.55. | H | Br | O-(4-F-C₆H₄) |
| I.1.56. | H | Br | O-(4-Cl-C₆H₄) |
| I.1.57. | H | I | H |
| I.1.58. | H | I | F |
| I.1.59. | H | I | Cl |
| I.1.60. | H | I | NO₂ |
| I.1.61. | H | I | CH₃ |
| I.1.62. | H | I | OCH₃ |
| I.1.63. | H | I | OCH₂CF₃ |
| I.1.64. | H | I | SCH₃ |
| I.1.65. | H | I | SCH₂CH₂N(CH₃)₂ |
| I.1.66. | H | I | SO₂N(CH₃)₂ |
| I.1.67. | H | I | S-C₆H₅ |
| I.1.68. | H | I | O-C₆H₅ |
| I.1.69. | H | I | O-(4-F-C₆H₄) |
| I.1.70. | H | I | O-(4-Cl-C₆H₄) |
| I.1.71. | H | CH₃ | H |
| I.1.72. | H | CH₃ | F |
| I.1.73. | H | CH₃ | Cl |
| I.1.74. | H | CH₃ | NO₂ |
| I.1.75. | H | CH₃ | CH₃ |
| I.1.76. | H | CH₃ | OCH₃ |
| I.1.77. | H | CH₃ | OCH₂CF₃ |
| I.1.78. | H | CH₃ | SCH₃ |
| I.1.79. | H | CH₃ | SCH₂CH₂N(CH₃)₂ |
| I.1.80. | H | CH₃ | SO₂N(CH₃)₂ |
| I.1.81. | H | CH₃ | S-C₆H₅ |
| I.1.82. | H | CH₃ | O-C₆H₅ |
| I.1.83. | H | CH₃ | O-(4-F-C₆H₄) |
| I.1.84. | H | CH₃ | O-(4-Cl-C₆H₄) |
| I.1.85. | H | OCH₃ | H |
| I.1.86. | H | OCH₃ | F |
| I.1.87. | H | OCH₃ | Cl |
| I.1.88. | H | OCH₃ | NO₂ |
| I.1.89. | H | OCH₃ | CH₃ |
| I.1.90. | H | OCH₃ | OCH₃ |
| I.1.91. | H | OCH₃ | OCH₂CF₃ |
| I.1.92. | H | OCH₃ | SCH₃ |
| I.1.93. | H | OCH₃ | SCH₂CH₂N(CH₃)₂ |
| I.1.94. | H | OCH₃ | SO₂N(CH₃)₂ |
| I.1.95. | H | OCH₃ | S-C₆H₅ |
| I.1.96. | H | OCH₃ | O-C₆H₅ |
| I.1.97. | H | OCH₃ | O-(4-F-C₆H₄) |
| I.1.98. | H | OCH₃ | O-(4-Cl-C₆H₄) |
| I.1.99. | H | N(CH₃)₂ | H |
| I.1.100 | H | N(CH₃)₂ | F |
| I.1.101 | H | N(CH₃)₂ | Cl |
| I.1.102 | H | N(CH₃)₂ | NO₂ |
| I.1.103 | H | N(CH₃)₂ | CH₃ |
| I.1.104 | H | N(CH₃)₂ | OCH₃ |
| I.1.105 | H | N(CH₃)₂ | OCH₂CF₃ |
| I.1.106 | H | N(CH₃)₂ | SCH₃ |
| I.1.107 | H | N(CH₃)₂ | SCH₂CH₂N(CH₃)₂ |
| I.1.108 | H | N(CH₃)₂ | SO₂N(CH₃)₂ |
| I.1.109 | H | N(CH₃)₂ | S-C₆H₅ |
| I.1.110 | H | N(CH₃)₂ | O-C₆H₅ |
| I.1.111 | H | N(CH₃)₂ | O-(4-F-C₆H₄) |
| I.1.112 | H | N(CH₃)₂ | O-(4-Cl-C₆H₄) |
| I.1.113 | H | C₆H₅ | H |
| I.1.114 | H | C₆H₅ | F |
| I.1.115 | H | C₆H₅ | Cl |
| I.1.116 | H | C₆H₅ | NO₂ |
| I.1.117 | H | C₆H₅ | CH₃ |
| I.1.118 | H | C₆H₅ | OCH₃ |
| I.1.119 | H | C₆H₅ | OCH₂CF₃ |
| I.1.120 | H | C₆H₅ | SCH₃ |
| I.1.121 | H | C₆H₅ | SCH₂CH₂N(CH₃)₂ |
| I.1.122 | H | C₆H₅ | SO₂N(CH₃)₂ |
| I.1.123 | H | C₆H₅ | S-C₆H₅ |
| I.1.124 | H | C₆H₅ | O-C₆H₅ |
| I.1.125 | H | C₆H₅ | O-(4-F-C₆H₄) |
| I.1.126 | H | C₆H₅ | O-(4-Cl-C₆H₄) |
| I.1.127 | Cl | H | H |
| I.1.128 | Cl | H | F |
| I.1.129 | Cl | H | Cl |
| I.1.130 | Cl | H | NO₂ |
| I.1.131 | Cl | H | CH₃ |
| I.1.132 | Cl | H | OCH₃ |
| I.1.133 | Cl | H | OCH₂CF₃ |
| I.1.134 | Cl | H | SCH₃ |
| I.1.135 | Cl | H | SCH₂CH₂N(CH₃)₂ |
| I.1.136 | Cl | H | SO₂N(CH₃)₂ |
| I.1.137 | Cl | H | S-C₆H₅ |
| I.1.138 | Cl | H | O-C₆H₅ |
| I.1.139 | Cl | H | O-(4-F-C₆H₄) |
| I.1.140 | Cl | H | O-(4-Cl-C₆H₄) |
| I.1.141 | Cl | F | H |
| I.1.142 | Cl | F | F |
| I.1.143 | Cl | F | Cl |
| I.1.144 | Cl | F | NO₂ |
| I.1.145 | Cl | F | CH₃ |
| I.1.146 | Cl | F | OCH₃ |
| I.1.147 | Cl | F | OCH₂CF₃ |
| I.1.148 | Cl | F | SCH₃ |
| I.1.149 | Cl | F | SCH₂CH₂N(CH₃)₂ |
| I.1.150 | Cl | F | SO₂N(CH₃)₂ |
| I.1.151 | Cl | F | S-C₆H₅ |
| I.1.152 | Cl | F | O-C₆H₅ |
| I.1.153 | Cl | F | O-(4-F-C₆H₄) |
| I.1.154 | Cl | F | O-(4-Cl-C₆H₄) |
| I.1.155 | Cl | Cl | H |
| I.1.156 | Cl | Cl | F |
| I.1.157 | Cl | Cl | Cl |
| I.1.158 | Cl | Cl | NO₂ |
| I.1.159 | Cl | Cl | CH₃ |
| I.1.160 | Cl | Cl | OCH₃ |
| I.1.161 | Cl | Cl | OCH₂CF₃ |
| I.1.162 | Cl | Cl | SCH₃ |
| I.1.163 | Cl | Cl | SCH₂CH₂N(CH₃)₂ |
| I.1.164 | Cl | Cl | SO₂N(CH₃)₂ |
| I.1.165 | Cl | Cl | S-C₆H₅ |
| I.1.166 | Cl | Cl | O-C₆H₅ |
| I.1.167 | Cl | Cl | O-(4-F-C₆H₄) |
| I.1.168 | Cl | Cl | O-(4-Cl-C₆H₄) |
| I.1.169 | Cl | Br | H |
| I.1.170 | Cl | Br | F |
| I.1.171 | Cl | Br | Cl |
| I.1.172 | Cl | Br | NO₂ |
| I.1.173 | Cl | Br | CH₃ |
| I.1.174 | Cl | Br | OCH₃ |
| I.1.175 | Cl | Br | OCH₂CF₃ |
| I.1.176 | Cl | Br | SCH₃ |
| I.1.177 | Cl | Br | SCH₂CH₂N(CH₃)₂ |
| I.1.178 | Cl | Br | SO₂N(CH₃)₂ |
| I.1.179 | Cl | Br | S-C₆H₅ |
| I.1.180 | Cl | Br | O-C₆H₅ |
| I.1.181 | Cl | Br | O-(4-F-C₆H₄) |
| I.1.182 | Cl | Br | O-(4-Cl-C₆H₄) |
| I.1.183 | Cl | I | H |
| I.1.184 | Cl | I | F |
| I.1.185 | Cl | I | Cl |
| I.1.186 | Cl | I | NO₂ |
| I.1.187 | Cl | I | CH₃ |
| I.1.188 | Cl | I | OCH₃ |
| I.1.189 | Cl | I | OCH₂CF₃ |
| I.1.190 | Cl | I | SCH₃ |
| I.1.191 | Cl | I | SCH₂CH₂N(CH₃)₂ |
| I.1.192 | Cl | I | SO₂N(CH₃)₂ |
| I.1.193 | Cl | I | S-C₆H₅ |
| I.1.194 | Cl | I | O-C₆H₅ |
| I.1.195 | Cl | I | O-(4-F-C₆H₄) |
| I.1.196 | Cl | I | O-(4-Cl-C₆H₄) |
| I.1.197 | Cl | CH₃ | H |
| I.1.198 | Cl | CH₃ | F |
| I.1.199 | Cl | CH₃ | Cl |
| I.1.200 | Cl | CH₃ | NO₂ |
| I.1.201 | Cl | CH₃ | CH₃ |
| I.1.202 | Cl | CH₃ | OCH₃ |
| I.1.203 | Cl | CH₃ | OCH₂CF₃ |
| I.1.204 | Cl | CH₃ | SCH₃ |
| I.1.205 | Cl | CH₃ | SCH₂CH₂N(CH₃)₂ |
| I.1.206 | Cl | CH₃ | SO₂N(CH₃)₂ |
| I.1.207 | Cl | CH₃ | S-C₆H₅ |
| I.1.208 | Cl | CH₃ | O-C₆H₅ |
| I.1.209 | Cl | CH₃ | O-(4-F-C₆H₄) |
| I.1.210 | Cl | CH₃ | O-(4-Cl-C₆H₄) |
| I.1.211 | Cl | OCH₃ | H |
| I.1.212 | Cl | OCH₃ | F |
| I.1.213 | Cl | OCH₃ | Cl |
| I.1.214 | Cl | OCH₃ | NO₂ |
| I.1.215 | Cl | OCH₃ | CH₃ |
| I.1.216 | Cl | OCH₃ | OCH₃ |
| I.1.217 | Cl | OCH₃ | OCH₂CF₃ |
| I.1.218 | Cl | OCH₃ | SCH₃ |
| I.1.219 | Cl | OCH₃ | SCH₂CH₂N(CH₃)₂ |
| I.1.220 | Cl | OCH₃ | SO₂N(CH₃)₂ |
| I.1.221 | Cl | OCH₃ | S-C₆H₅ |
| I.1.222 | Cl | OCH₃ | O-C₆H₅ |
| I.1.223 | Cl | OCH₃ | O-(4-F-C₆H₄) |
| I.1.224 | Cl | OCH₃ | O-(4-Cl-C₆H₄) |
| I.1.225 | Cl | N(CH₃)₂ | H |
| I.1.226 | Cl | N(CH₃)₂ | F |
| I.1.227 | Cl | N(CH₃)₂ | Cl |
| I.1.228 | Cl | N(CH₃)₂ | NO₂ |
| I.1.229 | Cl | N(CH₃)₂ | CH₃ |
| I.1.230 | Cl | N(CH₃)₂ | OCH₃ |
| I.1.231 | Cl | N(CH₃)₂ | OCH₂CF₃ |
| I.1.232 | Cl | N(CH₃)₂ | SCH₃ |
| I.1.233 | Cl | N(CH₃)₂ | SCH₂CH₂N(CH₃)₂ |
| I.1.234 | Cl | N(CH₃)₂ | SO₂N(CH₃)₂ |
| I.1.235 | Cl | N(CH₃)₂ | S-C₆H₅ |
| I.1.236 | Cl | N(CH₃)₂ | O-C₆H₅ |
| I.1.237 | Cl | N(CH₃)₂ | O-(4-F-C₆H₄) |
| I.1.238 | Cl | N(CH₃)₂ | O-(4-Cl-C₆H₄) |
| I.1.239 | Cl | C₆H₅ | H |
| I.1.240 | Cl | C₆H₅ | F |
| I.1.241 | Cl | C₆H₅ | Cl |
| I.1.242 | Cl | C₆H₅ | NO₂ |
| I.1.243 | Cl | C₆H₅ | CH₃ |
| I.1.244 | Cl | C₆H₅ | OCH₃ |
| I.1.245 | Cl | C₆H₅ | OCH₂CF₃ |
| I.1.246 | Cl | C₆H₅ | SCH₃ |
| I.1.247 | Cl | C₆H₅ | SCH₂CH₂N(CH₃)₂ |
| I.1.248 | Cl | C₆H₅ | SO₂N(CH₃)₂ |
| I.1.249 | Cl | C₆H₅ | S-C₆H₅ |
| I.1.250 | Cl | C₆H₅ | O-C₆H₅ |
| I.1.251 | Cl | C₆H₅ | O-(4-F-C₆H₄) |
| I.1.252 | Cl | C₆H₅ | O-(4-Cl-C₆H₄) |
| I.1.253 | CH₃ | H | H |
| I.1.254 | CH₃ | H | F |
| I.1.255 | CH₃ | H | Cl |
| I.1.256 | CH₃ | H | NO₂ |
| I.1.257 | CH₃ | H | CH₃ |
| I.1.258 | CH₃ | H | OCH₃ |
| I.1.259 | CH₃ | H | OCH₂CF₃ |
| I.1.260 | CH₃ | H | SCH₃ |
| I.1.261 | CH₃ | H | SCH₂CH₂N(CH₃)₂ |
| I.1.262 | CH₃ | H | SO₂N(CH₃)₂ |
| I.1.263 | CH₃ | H | S-C₆H₅ |
| I.1.264 | CH₃ | H | O-C₆H₅ |
| I.1.265 | CH₃ | H | O-(4-F-C₆H₄) |
| I.1.266 | CH₃ | H | O-(4-Cl-C₆H₄) |
| I.1.267 | CH₃ | F | H |
| I.1.268 | CH₃ | F | F |
| I.1.269 | CH₃ | F | Cl |
| I.1.270 | CH₃ | F | NO₂ |
| I.1.271 | CH₃ | F | CH₃ |
| I.1.272 | CH₃ | F | OCH₃ |
| I.1.273 | CH₃ | F | OCH₂CF₃ |
| I.1.274 | CH₃ | F | SCH₃ |
| I.1.275 | CH₃ | F | SCH₂CH₂N(CH₃)₂ |
| I.1.276 | CH₃ | F | SO₂N(CH₃)₂ |
| I.1.277 | CH₃ | F | S-C₆H₅ |
| I.1.278 | CH₃ | F | O-C₆H₅ |
| I.1.279 | CH₃ | F | O-(4-F-C₆H₄) |
| I.1.280 | CH₃ | F | O-(4-Cl-C₆H₄) |
| I.1.281 | CH₃ | Cl | H |
| I.1.282 | CH₃ | Cl | F |
| I.1.283 | CH₃ | Cl | Cl |
| I.1.284 | CH₃ | Cl | NO₂ |
| I.1.285 | CH₃ | Cl | CH₃ |
| I.1.286 | CH₃ | Cl | OCH₃ |
| I.1.287 | CH₃ | Cl | OCH₂CF₃ |
| I.1.288 | CH₃ | Cl | SCH₃ |
| I.1.289 | CH₃ | Cl | SCH₂CH₂N(CH₃)₂ |
| I.1.290 | CH₃ | Cl | SO₂N(CH₃)₂ |
| I.1.291 | CH₃ | Cl | S-C₆H₅ |
| I.1.292 | CH₃ | Cl | O-C₆H₅ |
| I.1.293 | CH₃ | Cl | O-(4-F-C₆H₄) |
| I.1.294 | CH₃ | Cl | O-(4-Cl-C₆H₄) |
| I.1.295 | CH₃ | Br | H |
| I.1.296 | CH₃ | Br | F |
| I.1.297 | CH₃ | Br | Cl |
| I.1.298 | CH₃ | Br | NO₂ |
| I.1.299 | CH₃ | Br | CH₃ |
| I.1.300 | CH₃ | Br | OCH₃ |
| I.1.301 | CH₃ | Br | OCH₂CF₃ |
| I.1.302 | CH₃ | Br | SCH₃ |
| I.1.303 | CH₃ | Br | SCH₂CH₂N(CH₃)₂ |
| I.1.304 | CH₃ | Br | SO₂N(CH₃)₂ |
| I.1.305 | CH₃ | Br | S-C₆H₅ |
| I.1.306 | CH₃ | Br | O-C₆H₅ |
| I.1.307 | CH₃ | Br | O-(4-F-C₆H₄) |
| I.1.308 | CH₃ | Br | O-(4-Cl-C₆H₄) |
| I.1.309 | CH₃ | I | H |
| I.1.310 | CH₃ | I | F |
| I.1.311 | CH₃ | I | Cl |
| I.1.312 | CH₃ | I | NO₂ |
| I.1.313 | CH₃ | I | CH₃ |
| I.1.314 | CH₃ | I | OCH₃ |
| I.1.315 | CH₃ | I | OCH₂CF₃ |
| I.1.316 | CH₃ | I | SCH₃ |
| I.1.317 | CH₃ | I | SCH₂CH₂N(CH₃)₂ |
| I.1.318 | CH₃ | I | SO₂N(CH₃)₂ |
| I.1.319 | CH₃ | I | S-C₆H₅ |
| I.1.320 | CH₃ | I | O-C₆H₅ |
| I.1.321 | CH₃ | I | O-(4-F-C₆H₄) |
| I.1.322 | CH₃ | I | O-(4-Cl-C₆H₄) |
| I.1.323 | CH₃ | CH₃ | H |
| I.1.324 | CH₃ | CH₃ | F |
| I.1.325 | CH₃ | CH₃ | Cl |
| I.1.326 | CH₃ | CH₃ | NO₂ |
| I.1.327 | CH₃ | CH₃ | CH₃ |
| I.1.328 | CH₃ | CH₃ | OCH₃ |
| I.1.329 | CH₃ | CH₃ | OCH₂CF₃ |
| I.1.330 | CH₃ | CH₃ | SCH₃ |
| I.1.331 | CH₃ | CH₃ | SCH₂CH₂N(CH₃)₂ |
| I.1.332 | CH₃ | CH₃ | SO₂N(CH₃)₂ |
| I.1.333 | CH₃ | CH₃ | S-C₆H₅ |
| I.1.334 | CH₃ | CH₃ | O-C₆H₅ |
| I.1.335 | CH₃ | CH₃ | O-(4-F-C₆H₄) |
| I.1.336 | CH₃ | CH₃ | O-(4-Cl-C₆H₄) |
| I.1.337 | CH₃ | OCH₃ | H |
| I.1.338 | CH₃ | OCH₃ | F |
| I.1.339 | CH₃ | OCH₃ | Cl |
| I.1.340 | CH₃ | OCH₃ | NO₂ |
| I.1.341 | CH₃ | OCH₃ | CH₃ |
| I.1.342 | CH₃ | OCH₃ | OCH₃ |
| I.1.343 | CH₃ | OCH₃ | OCH₂CF₃ |
| I.1.344 | CH₃ | OCH₃ | SCH₃ |
| I.1.345 | CH₃ | OCH₃ | SCH₂CH₂N(CH₃)₂ |
| I.1.346 | CH₃ | OCH₃ | SO₂N(CH₃)₂ |
| I.1.347 | CH₃ | OCH₃ | S-C₆H₅ |
| I.1.348 | CH₃ | OCH₃ | O-C₆H₅ |
| I.1.349 | CH₃ | OCH₃ | O-(4-F-C₆H₄) |
| I.1.350 | CH₃ | OCH₃ | O-(4-Cl-C₆H₄) |
| I.1.351 | CH₃ | N(CH₃)₂ | H |
| I.1.352 | CH₃ | N(CH₃)₂ | F |
| I.1.353 | CH₃ | N(CH₃)₂ | Cl |
| I.1.354 | CH₃ | N(CH₃)₂ | NO₂ |
| I.1.355 | CH₃ | N(CH₃)₂ | CH₃ |
| I.1.356 | CH₃ | N(CH₃)₂ | OCH₃ |
| I.1.357 | CH₃ | N(CH₃)₂ | OCH₂CF₃ |
| I.1.358 | CH₃ | N(CH₃)₂ | SCH₃ |
| I.1.359 | CH₃ | N(CH₃)₂ | SCH₂CH₂N(CH₃)₂ |
| I.1.360 | CH₃ | N(CH₃)₂ | SO₂N(CH₃)₂ |
| I.1.361 | CH₃ | N(CH₃)₂ | S-C₆H₅ |
| I.1.362 | CH₃ | N(CH₃)₂ | O-C₆H₅ |
| I.1.363 | CH₃ | N(CH₃)₂ | O-(4-F-C₆H₄) |
| I.1.364 | CH₃ | N(CH₃)₂ | O-(4-Cl-C₆H₄) |
| I.1.365 | CH₃ | C₆H₅ | H |
| I.1.366 | CH₃ | C₆H₅ | F |
| I.1.367 | CH₃ | C₆H₅ | Cl |
| I.1.368 | CH₃ | C₆H₅ | NO₂ |
| I.1.369 | CH₃ | C₆H₅ | CH₃ |
| I.1.370 | CH₃ | C₆H₅ | OCH₃ |
| I.1.371 | CH₃ | C₆H₅ | OCH₂CF₃ |
| I.1.372 | CH₃ | C₆H₅ | SCH₃ |
| I.1.373 | CH₃ | C₆H₅ | SCH₂CH₂N(CH₃)₂ |
| I.1.374 | CH₃ | C₆H₅ | SO₂N(CH₃)₂ |
| I.1.375 | CH₃ | C₆H₅ | S-C₆H₅ |
| I.1.376 | CH₃ | C₆H₅ | O-C₆H₅ |
| I.1.377 | CH₃ | C₆H₅ | O-(4-F-C₆H₄) |
| I.1.378 | CH₃ | C₆H₅ | O-(4-Cl-C₆H₄) |

Also preferred are the acylhydrazides of formula 1.2, particularly preferred the acylhydrazides of formulae I.2.1 to 1.2.378, which differ from the corresponding acylhydrazides of formulae I.1.1 to I.1.378 only in that R⁸ is CH₃:

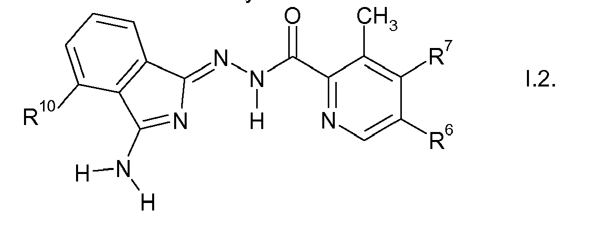

Also preferred are the acylhydrazides of formula 1.3, particularly preferred the acylhydrazides of formulae I.3.1 to I.3.378, which differ from the corresponding acylhydrazides of formulae I.1.1 to I.1.378 only in that R¹¹ is F:

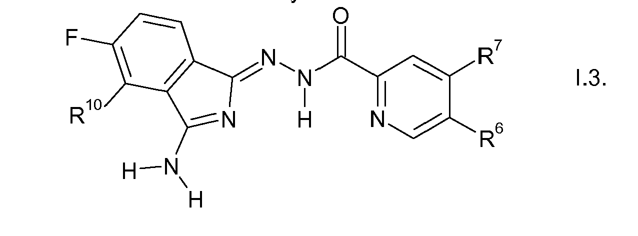

Also preferred are the acylhydrazides of formula 1.4, particularly preferred the acylhydrazides of formulae I.4.1 to I.4.378, which differ from the corresponding acylhydrazides of formulae I.1.1 to I.1.378 only in that R⁸ is CH₃ and R¹¹ is F:

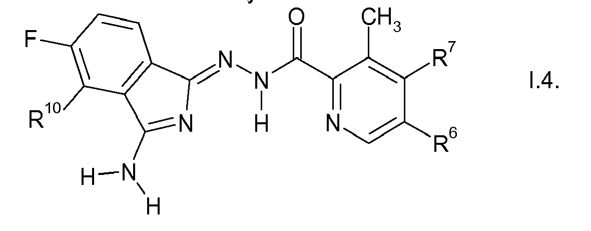

Also preferred are the acylhydrazides of formula 1.5, particularly preferred the acylhydrazides of formulae I.5.1 to I.5.378, which differ from the corresponding acylhydrazides of formulae I.1.1 to I.1.378 only in that R¹² is F:

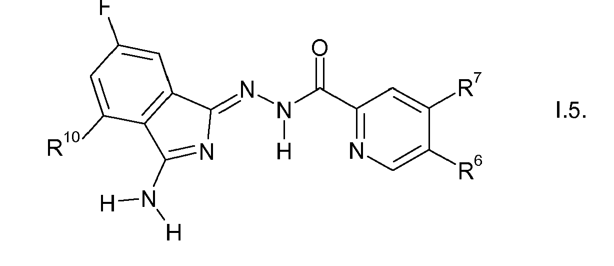

Also preferred are the acylhydrazides of formula 1.6, particularly preferred the acylhydrazides of formulae I.6.1 to 1.6.378, which differ from the corresponding acylhydrazides of formulae I.1.1 to I.1.378 only in that R⁸ is CH₃ and R¹² is F:

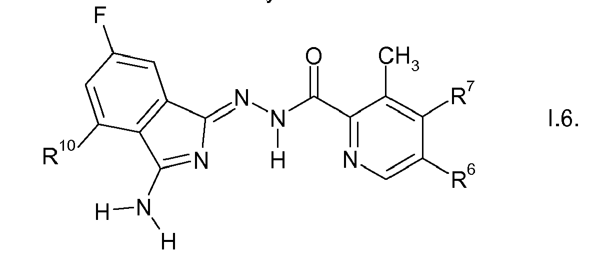

Also preferred are the acylhydrazides of formula 1.7, particularly preferred the acylhydrazides of formulae I.7.1 to I.7.378, which differ from the corresponding acylhydrazides of formulae I.1.1 to I.1.378 only in that R¹³ is F:

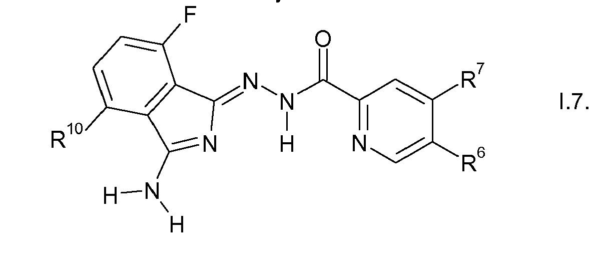

Also preferred are the acylhydrazides of formula 1.8, particularly preferred the acylhydrazides of formulae I.8.1 to I.8.378, which differ from the corresponding acylhydrazides of formulae I.1.1 to I.1.378 only in that R⁸ is CH₃ and R¹³ is F:

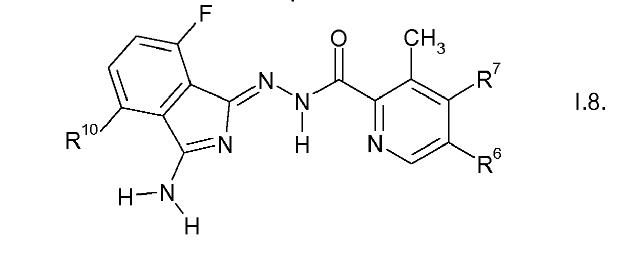

Also preferred are the acylhydrazides of formula 1.9, particularly preferred the acylhydrazides of formulae I.9.1 to I.9.378, which differ from the corresponding acylhydrazides of formulae I.1.1 to I.1.378 only in that R¹¹ and R¹² are F:

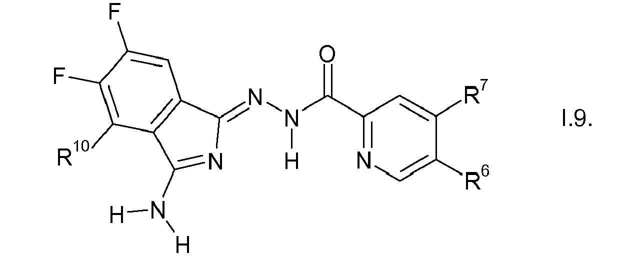

Also preferred are the acylhydrazides of formula I.10, particularly preferred the acylhydrazides of formulae I.10.1 to I.10.378, which differ from the corresponding acylhydrazides of formulae I.1.1 to I.1.378 only in that R⁸ is CH₃, and R¹¹ and R¹² are F:

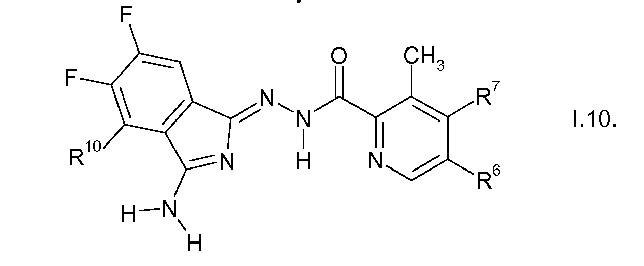

Also preferred are the acylhydrazides of formula I.11, particularly preferred the acylhydrazides of formulae I.11.1 to I.11.378, which differ from the corresponding acylhydrazides of formulae I.1.1 to I.1.378 only in that R¹¹ and R¹³ are F:

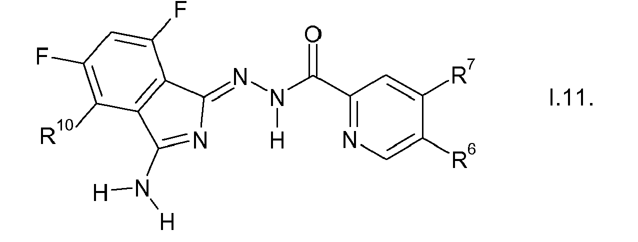

Also preferred are the acylhydrazides of formula I.12, particularly preferred the acylhydrazides of formulae I.12.1 to I.12.378, which differ from the corresponding acylhydrazides of formulae I.1.1 to I.1.378 only in that R⁸ is CH₃, R¹¹ and R¹³ are F:

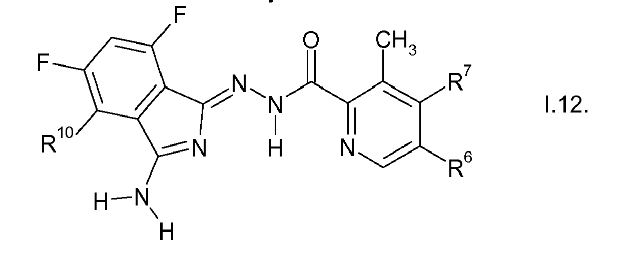

Also preferred are the acylhydrazides of formula I.13, particularly preferred the acylhydrazides of formulae I.13.1 to I.13.378, which differ from the corresponding acylhydrazides of formulae I.1.1 to I.1.378 only in that R¹² and R¹³ are F:

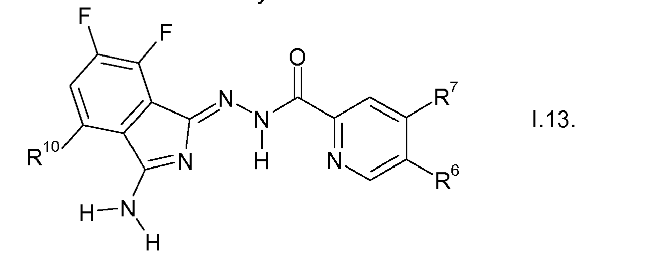

Also preferred are the acylhydrazides of formula 1.14, particularly preferred the acylhydrazides of formulae I.14.1 to 1.14.378, which differ from the corresponding acylhydrazides of formulae I.1.1 to I.1.378 only in that R⁸ is CH₃, and R¹² and R¹³ are F:

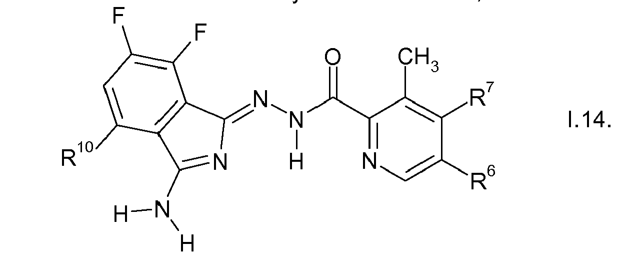

Also preferred are the acylhydrazides of formula I.15, particularly preferred the acylhydrazides of formulae I.15.1 to I.15.378, which differ from the corresponding acylhydrazides of formulae I.1.1 to I.1.378 only in that R¹¹, R¹² and R¹³ are F:

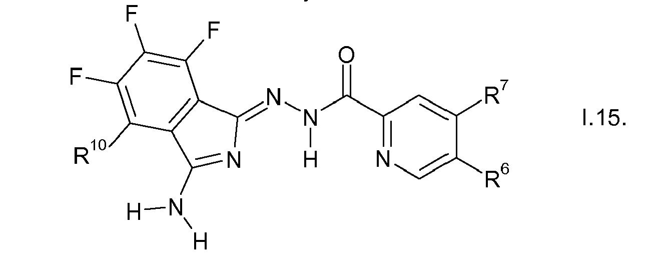

Also preferred are the acylhydrazides of formula I.16, particularly preferred the acylhydrazides of formulae I.16.1 to I.16.378, which differ from the corresponding acylhydrazides of formulae I.1.1 to I.1.378 only in that R⁸ is CH₃, and R¹¹, R¹² and R¹³ are F:

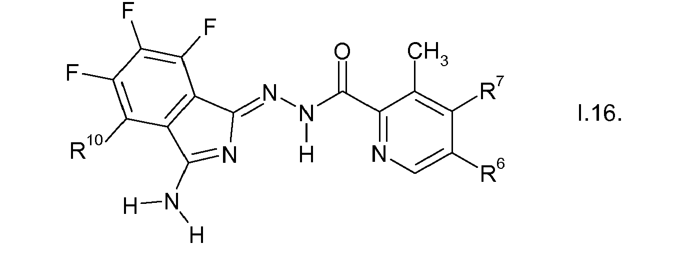

The acylhydrazides of formula I according to the invention can be prepared by standard processes of organic chemistry, for example by the following processes:

### Process A)

The acylhydrazides of formula I, wherein R⁴ is hydrogen can be obtained by direct cyclisation of an cyclyl-1,2-dinitrile of formula II with an hydrazide of formula III:

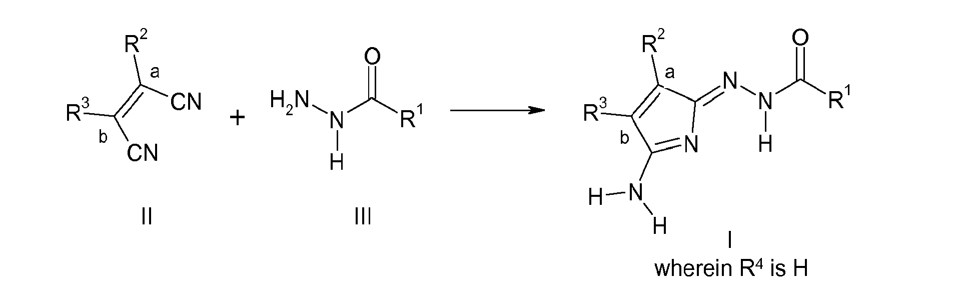

The cyclisation of the cyclyl-1,2-dinitrile of formula II with the hydrazide of formula III is usually carried out at from 20°C to the boiling point of the reaction mixture, preferably at from 50°C to 150°C, particularly preferably at from 80°C to 150°C, in an inert organic solvent optionally in the presence of a base (Eur. J. of Org. Chem. (12) 2006, 2833-2842).

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, ketones such as acetone, methyl ethyl ketone, diethyl ketone and tert-butyl methyl ketone, alkoholes such as methanol, ethanol, n-propanol, isopropanol, n-butanol, isobutanol and tert.-butanol, as well as dimethylsulfoxide, dimethylformamide and N,N-dimethylacetamide or N.methylpyrrolidone. Particular preference is given to alkoholes such as methanol, ethanol, n-propanol, isopropanol, n-butanol, isobutanol and tert.-butanol. It is also possible to use mixtures of the solvents mentioned. Suitable bases are, in general inorganic compounds such as alkali metal and alkaline earth metal hydroxides such as lithium hydroxide, sodium hydroxide, potassium hydroxide and calcium hydroxide, alkali metal and alkaline earth metal oxide such as lithium oxide, sodium oxide, calcium oxide and magnesium oxide, alkali metal and alkaline earth metal hydrides such as lithium hydride, sodium hydride, potassium hydride and calcium hydride, alkali metal amides such as lithium amide, sodium amide and potassium amide, alkali metal and alkaline earth metal carbonates such as lithium carbonate, potassium carbonate and calcium carbonate, as well as alkali metal bicarbonates such as sodium bicarbonate, alkyl magnesium halides such as methyl magnesium chloride as well as alkali metal and alkaline earth metal alkoxides such as sodium methoxide, sodium ethoxide, potassium ethoxide, potassium tert-butoxide, potassium tert-pentoxide and dimethoxymagnesium, and furthermore organic bases such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to alkali metal and alkaline earth metal alkoxides such as sodium methoxide, sodium ethoxide, potassium ethoxide, potassium tert-butoxide, potassium tert-pentoxide and dimethoxymagnesium.

The bases are generally employed in catalytic amounts however they can also be employed in equimolar amounts, in excess or, if appropriate, be used as solvent.

The reaction mixtures are worked up in a customary manner, for example by mixing with water, separation of the phases and, if appropriate, chromatographic purification of the crude product. Some of the intermediates and end products are obtained in the form of viscous oils, which can be purified or freed from volatile components under reduced pressure and at moderately elevated temperature. If the intermediates and the end products are obtained as solid, purification can also be carried out by recrystallisation or digestion.

### Process B)

As an alternative, the acylhydrazides of formula I can be obtained by first cyclizing the cyclyl-1,2-dinitrile of formula II with an amine IV, preferably ammonia, to give the corresponding 3-imino-3H-isoindol-1-ylamine of formula V, and then subsequently substituting one amino group with the corresponding hydrazide of formula III:

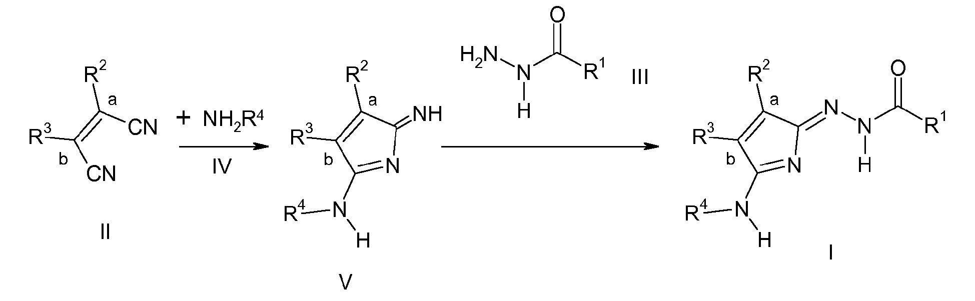

The cyclisation of the cyclyl-1,2-dinitrile of formula II with ammonia is usually carried out at from 0°C to the boiling point of the reaction mixture, preferably at from 20°C to 150°C, particularly preferably at from 20°C to 100°C, in an inert organic solvent optionally in the presence of a base (Tetrahedron Letters 2003, 44 (9), 1967 - 1970).

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, ketones such as acetone, methyl ethyl ketone, diethyl ketone and tert-butyl methyl ketone, alkoholes such as methanol, ethanol, n-propanol, isopropanol, n-butanol and tert.-butanol, as well as dimethylsulfoxide, dimethylformamide and N,N-dimethylacetamide or N.methylpyrrolidone. Particular preference is given to alkoholes such as methanol, ethanol, n-propanol, isopropanol, n-butanol and tert.-butanol. It is also possible to use mixtures of the solvents mentioned.

Suitable bases are, in general inorganic compounds such as alkali metal and alkaline earth metal hydroxides such as lithium hydroxide, sodium hydroxide, potassium hydroxide and calcium hydroxide, alkali metal and alkaline earth metal oxide such as lithium oxide, sodium oxide, calcium oxide and magnesium oxide, alkali metal and alkaline earth metal hydrides such as lithium hydride, sodium hydride, potassium hydride and calcium hydride, alkali metal amides such as lithium amide, sodium amide and potassium amide, alkali metal and alkaline earth metal carbonates such as lithium carbonate, potassium carbonate and calcium carbonate, as well as alkali metal bicarbonates such as sodium bicarbonate, alkyl magnesium halides such as methyl magnesium chloride as well as alkali metal and alkaline earth metal alkoxides such as sodium methoxide, sodium ethoxide, potassium ethoxide, potassium tert-butoxide, potassium tert-pentoxide and dimethoxymagnesium, and furthermore organic bases such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to alkali metal and alkaline earth metal alkoxides such as sodium methoxide, sodium ethoxide, potassium ethoxide, potassium tert-butoxide, potassium tert-pentoxide and dimethoxymagnesium. The bases are generally employed in catalytic amounts however they can also be employed in equimolar amounts, in excess or, if appropriate, be used as solvent.

Work up can be carried out in a known manner. It is also possible to use the crude reaction mixture containing the 3-imino-3H-isoindol-1-ylamine of formula V within the next step.

The subsequent substitution of one amino group of the 3-imino-3H-isoindol-1-ylamine of formula V with the hydrazide III is usually carried out at from 20°C to the boiling point of the reaction mixture, preferably at from 20°C to 150°C, particularly preferably at from 50°C to 150°C, in an inert organic solvent optionally in the presence of a base (Eur. J. of Org. Chem. (12) 2006, 2833-2842)

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, ketones such as acetone, methyl ethyl ketone, diethyl ketone and tert-butyl methyl ketone, alkoholes such as methanol, ethanol, n-propanol, isopropanol, n-butanol and tert.-butanol, as well as dimethylsulfoxide, dimethylformamide and N,N-dimethylacetamide or N.methylpyrrolidone. Particular preference is given to alkoholes such as methanol, ethanol, n-propanol, isopropanol, n-butanol and tert.-butanol. It is also possible to use mixtures of the solvents mentioned.

Suitable bases are, in general inorganic compounds such as alkali metal and alkaline earth metal hydroxides such as lithium hydroxide, sodium hydroxide, potassium hydroxide and calcium hydroxide, alkali metal and alkaline earth metal oxide such as lithium oxide, sodium oxide, calcium oxide and magnesium oxide, alkali metal and alkaline earth metal hydrides such as lithium hydride, sodium hydride, potassium hydride and calcium hydride, alkali metal amides such as lithium amide, sodium amide and potassium amide, alkali metal and alkaline earth metal carbonates such as lithium carbonate, potassium carbonate and calcium carbonate, as well as alkali metal bicarbonates such as sodium bicarbonate, alkyl magnesium halides such as methyl magnesium chloride as well as alkali metal and alkaline earth metal alkoxides such as sodium methoxide, sodium ethoxide, potassium ethoxide, potassium tert-butoxide, potassium tert-pentoxide and dimethoxymagnesium, and furthermore organic bases, such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to alkali metal and alkaline earth metal alkoxides such as sodium methoxide, sodium ethoxide, potassium ethoxide, potassium tert-butoxide, potassium tert-pentoxide and dimethoxymagnesium. The bases are generally employed in catalytic amounts however they can also be employed in equimolar amounts, in excess or, if appropriate, be used as solvent.

Work up can be carried out in a known manner.

### Process C)

Alternatively the acylhydrazides of formula I, wherein R⁴ is hydrogen, can be obtained by first cyclizing the cyclyl-1,2-dinitrile of formula II with hydrazine to give the corresponding 3-hydrazono-3H-isoindol-1-ylamine of formula VI, which is then acylated with an carbonyl compound of formula VII:

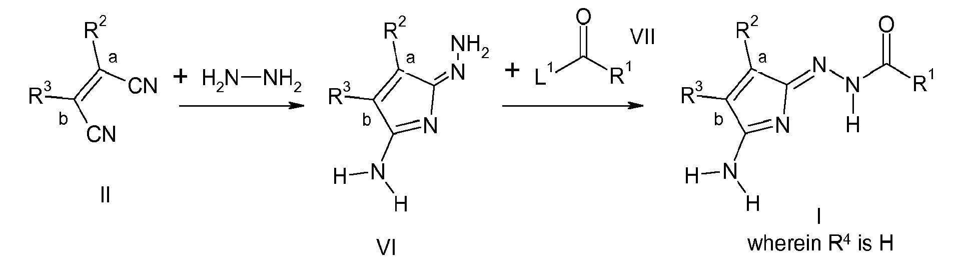

L¹ stands for a nucleophilically displaceable leaving group such as halogen, hydroxy, C₁-C₆-alkoxy, C₁-C₆-alkylcarbonyloxy, C₁-C₆-alkylamino-iminocarbonyloxy, C₁-C₆-alkylsulfonyl, C₁-C₆-haloalkylsulfonyl, C₁-C₆-alkylsulfonyloxy, C₁-C₆-haloalkylsulfonyloxy, arylsulfonyl, arylsulfonyloxy or trialkylammonium.

The cyclisation of the cyclyl-1,2-dinitrile of formula **II** with hydrazine is usually carried out at from 0°C to the boiling point of the reaction mixture, preferably at from 0°C to 150°C, particularly preferably at from 0°C to 150°C, in an inert organic solvent optionally in the presence of a base (Eur. J. of Org. Chem. (12) 2006, 2833-2842).

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, ketones such as acetone, methyl ethyl ketone, diethyl ketone and tert-butyl methyl ketone, alkoholes such as methanol, ethanol, n-propanol, isopropanol, n-butanol and tert.-butanol, as well as dimethylsulfoxide, dimethylformamide and N,N-dimethylacetamide or N.methylpyrrolidone. Particular preference is given to alkoholes such as methanol, ethanol, n-propanol, isopropanol, n-butanol and tert.-butanol. It is also possible to use mixtures of the solvents mentioned.

Suitable bases are, in general inorganic compounds such as alkali metal and alkaline earth metal hydroxides such as lithium hydroxide, sodium hydroxide, potassium hydroxide and calcium hydroxide, alkali metal and alkaline earth metal oxide such as lithium oxide, sodium oxide, calcium oxide and magnesium oxide, alkali metal and alkaline earth metal hydrides such as lithium hydride, sodium hydride, potassium hydride and calcium hydride, alkali metal amides such as lithium amide, sodium amide and potassium amide, alkali metal and alkaline earth metal carbonates such as lithium carbonate, potassium carbonate and calcium carbonate, as well as alkali metal bicarbonates such as sodium bicarbonate, alkyl magnesium halides such as methyl magnesium chloride as well as alkali metal and alkaline earth metal alkoxides such as sodium methoxide, sodium ethoxide, potassium ethoxide, potassium tert-butoxide, potassium tert-pentoxide and dimethoxymagnesium, and furthermore organic bases, such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to alkali metal and alkaline earth metal alkoxides such as sodium methoxide, sodium ethoxide, potassium ethoxide, potassium tert-butoxide, potassium tert-pentoxide and dimethoxymagnesium. The bases are generally employed in catalytic amounts however they can also be employed in equimolar amounts, in excess or, if appropriate, be used as solvent.

Work up can be carried out in a known manner. It is also possible to use the crude reaction mixture containing the 3-hydrazono-3H-isoindol-1-ylamine of formula VI within the next step.

The subsequent acylation of the 3-hydrazono-3H-isoindol-1-ylamine of formula VI with with a carbonyl compound of formula VII is usually carried out at from 0°C to the boiling point of the reaction mixture, preferably at from 0°C to 150°C, particularly preferably at from 20°C to 150°C, in an inert organic solvent optionally in the presence of a base (Eur. J. of Org. Chem. (12) 2006, 2833-2842).

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, ketones such as acetone, methyl ethyl ketone, diethyl ketone and tert-butyl methyl ketone, alkoholes such as methanol, ethanol, n-propanol, isopropanol, n-butanol and tert.-butanol, as well as dimethylsulfoxide, dimethylformamide and N,N-dimethylacetamide or N.methylpyrrolidone, Particular preference is given to alkoholes such as methanol, ethanol, n-propanol, isopropanol, n-butanol and tert.-butanol. It is also possible to use mixtures of the solvents mentioned.

Suitable bases are, in general inorganic compounds such as alkali metal and alkaline earth metal hydroxides such as lithium hydroxide, sodium hydroxide, potassium hydroxide and calcium hydroxide, alkali metal and alkaline earth metal oxide such as lithium oxide, sodium oxide, calcium oxide and magnesium oxide, alkali metal and alkaline earth metal hydrides such as lithium hydride, sodium hydride, potassium hydride and calcium hydride, alkali metal amides such as lithium amide, sodium amide and potassium amide, alkali metal and alkaline earth metal carbonates such as lithium carbonate, potassium carbonate and calcium carbonate, as well as alkali metal bicarbonates such as sodium bicarbonate, alkyl magnesium halides such as methyl magnesium chloride as well as alkali metal and alkaline earth metal alkoxides such as sodium methoxide, sodium ethoxide, potassium ethoxide, potassium tert-butoxide, potassium tert-pentoxide and dimethoxymagnesium, and furthermore organic bases, such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to alkali metal and alkaline earth metal alkoxides such as sodium methoxide, sodium ethoxide, potassium ethoxide, potassium tert-butoxide, potassium tert-pentoxide and dimethoxymagnesium. The bases are generally employed in catalytic amounts, however they can also be employed in equimolar amounts, in excess or, if appropriate, be used as solvent.

Work up can be carried out in a known manner.

### Process D:

The acylhydrazides of formula I, wherein R⁴ is formyl or C₁-C₆-alkylcarbonyl can be obtained by acylation of the respective acylhydrazides of formula I, wherein R⁴ is hydrogen:

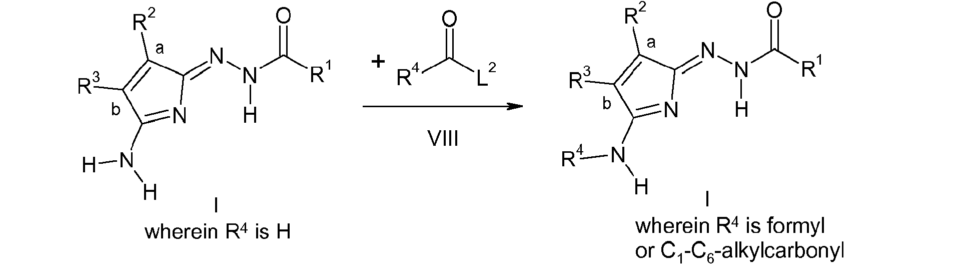

L² stand for a nucleophilically displaceable leaving group such as halogen, hydroxy, C₁-C₆-alkoxy, C₁-C₆-alkycarbonyloxy, phenoxy, C₁-C₆-alkylsulfonyloxy, C₁-C₆-haloalkylsulfonyloxy and arylsulfonyloxy; preferably for Cl, C₁-C₆-alkycarbonyloxy or phenoxy.

The acylation of the acylhydrazides of formula I, wherein R⁴ is hydrogen is usually carried out at from 0°C to the boiling point of the reaction mixture, preferably at from 0°C to 150°C, particularly preferably at from 20°C to 150°C optionally in an inert organic solvent optionally in the presence of a base.

Suitable acylation agents VIII are commercially available and are for example acetylchloride, acetic anhydride, phenolacetate, propionylchloride, propionic anhydride, phenolpropionate, butyrylchloride, butyric anhydride, phenolbutyrate, iso-butyrylchloride, iso-butyric anhydride and phenolisobutyrate.

Suitable solvents are aliphatic hydrocarbons such as pentane, hexane, cyclohexane and mixtures of C₅-C₈-alkanes, aromatic hydrocarbons such as tolene, o-, m- and p-xylene, halogenated hydrocarbons such asdichloromethane, 1,2-dichloroethane, chloroform and chlorobenzene, ethers such as diethyl ether, diisopropyl ether, tert.-butyl methylether, dioxane, anisole and tetrahydrofuran, nitriles such as acetonitrile and propionitrile, alkoholes such as methanol, ethanol, n-propanol, isopropanol, n-butanol and tert.-butanol, as well as dimethylsulfoxide, dimethylformamide and N,N-dimethylacetamide or N-methylpyrrolidone. Particular preference is given to tetrahydrofuran, acetonitrile and dimethylformamide. It is also possible to use mixtures of the solvents mentioned.

Suitable bases are, in general Inorganic compounds such as alkali metal and alkaline earth metal hydrides such as lithium hydride, sodium hydride, potassium hydride and calcium hydride, alkali metal and alkaline earth metal carbonates such as lithium carbonate, potassium carbonate and calcium carbonate, as well as alkali metal bicarbonates such as sodium bicarbonate, and furthermore organic bases, such as tertiary amines such as trimethylamine, triethylamine, diisopropylethylamine and N-methylpiperidine, pyridine, substituted pyridines such as collidine, lutidine, N-methylmorpholine and 4-dimethylaminopyridine and also bicyclic amines. Particular preference is given to triethylamine and potassium carbonate. The bases are generally employed in catalytic amounts, however they can also be employed in equimolar amounts, in excess or, if appropriate, be used as solvent.

In general, the educts are employed in equimolar amounts. It might be advantageous to employ an excess of acylating agent VIII.

Work up can be carried out in a known manner.

The cyclyl-1,2-dinitriles of formula II required for the preparation of acylhydrazides of formula I are known from the literature and can be prepared in accordance with the literature cited and/or are commercially available: transformation of substituted phtalicacids or heterocyclyl-1,2-diacids to the diamides and subsequent dehydration (J. of Het. Chem. 1995, 32(2), 495-498); substitution of the nitro or halogen-group of 3- or 4-nitro- or halogen-phthalo- or heterocyclyl-1,2-dinitrile by a nuclephile (Eur. J. of Org. Chem. 2000 (8), 1603-1607); transition-catalyzed coupling of a halogen- or sulfonyloxysubstituted benzo- or heterocyclyl-1,2-dinitrile with a carbon-nucleophile (Can. J. of Chem. 1995, 73(3), 435-443); nucleophilic substitution of a leaving group on an aromatic ring by one or two cyanides (J. of Het. Chem. 1991 28(5), 1357-63); alkylation of a hydroxyl-, amino- or thio-substituted benzo- or heterocyclyl-1,2-dinitrile (Bull. of the Chem. Soc. of Japan 1997, 70(11), 2693-2698).

The hydrazides of formula III required for the preparation of acylhydrazides of formula I are known from the literature (Organic Preparations and Procedures International, 29(1), 117-122; 1997 and can be prepared accordingly and/or are commercially available.

The carbonyl compounds of formula VII required for the preparation of acylhydrazides of formula I and IA are known from the literature (Chemical Communications, Cambridge, UK, (31), 3276-3278; 2007) and can be prepared accordingly and/or are commercially available.

The 3-imino-3H-isoindol-1-ylamines of formula V mentioned above are novel compounds and suitable intermediates for the preparation of the acylhydrazides of formula I according to the present invention.

Therefore the present invention also provides novel 3-imino-3H-isoindol-1-ylamines of formula V 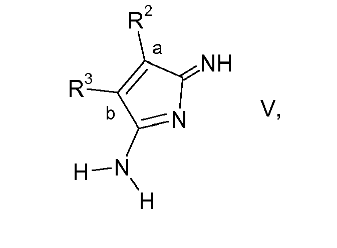 wherein R² and R³ correspond, either independently of one another or in combination with one another, to those of the variables of R² and R³ of the acylhydrazides of formula I as defined herein.

With respect to the variables R² and R³, the particularly preferred embodiments of the intermediate compounds V correspond, either independently of one another or in combination with one another, to those of the variables of R² and R³ of formula I as defined herein.

Special preference is given to the 3-imino-3H-isoindol-1-ylamines of formula V.A, which correspond to 3-imino-3H-isoindol-1-ylamines of formula V, wherein in case R² and R³ together with the carbon atoms (a) and (b) to which they are attached form a six-membered monocyclic cycle, preferably the cycle B1, said six-membered monocyclic cycle is partially or fully halogenated and/or is substituted by one to three substituents selected from the group as defined herein.

With regard to the preferred embodiments of the 3-imino-3H-isoindol-1-ylamines of formula V.A the preferred embodiments mentioned herein with regard to the acylhydrazides of formula I and the 3-imino-3H-isoindol-1-ylamines of formula V are fully applicable.

The 3-hydrazono-3H-isoindol-1-ylamines of formula VI mentioned above are novel compounds and suitable intermediates for the preparation of acylhydrazides of formula I and IA according to the present invention.

Therefore the present invention also provides novel 3-hydrazono-3H-isoindol-1-ylamines of formula VI 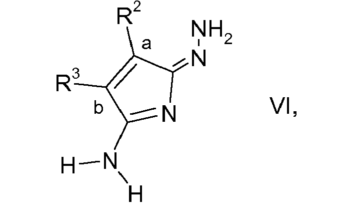 wherein R² and R³ correspond, either independently of one another or in combination with one another, to those of the variables of R² and R³ of the acylhydrazides of formula I as defined herein.

With respect to the variables R² and R³, the particularly preferred embodiments of the intermediate compounds VI correspond, either independently of one another or in combination with one another, to those of the variables of R² and R³ of formula I as defined herein.

Special preference is given to the 3-hydrazono-3H-isoindol-1-ylamines of formula VI.A, which correspond to 3-hydrazono-3H-isoindol-1-ylamines of formula VI, wherein in case R² and R³ together with the carbon atoms (a) and (b) to which they are attached form a six-membered monocyclic cycle, preferably the cycle B1, said six-membered monocyclic cycle is partially or fully halogenated and/or is substituted by one to three substituents selected from the group as defined herein.

With regard to the preferred embodiments of the 3-hydrazono-3H-isoindol-1-ylamines of formula VI.A the preferred embodiments mentioned herein with regard to the acylhydrazides of formula I and the 3-hydrazono-3H-isoindol-1-ylamines of formula VI are fully applicable.

The acylhydrazides of formula I are suitable as herbicides. They are suitable as such or as an appropriately formulated composition (herbicidal composition). As used in this application, the terms "formulated composition" and "herbicidal composition" are synonyms. The herbicidal compositions comprising the acylhydrazides of formula I control vegetation on non-crop areas very efficiently, especially at high rates of application. They act against broad-leaved weeds and grass weeds in crops such as wheat, rice, maize, soya and cotton without causing any significant damage to the crop plants. This effect is mainly observed at low rates of application.

Depending on the application method in question, the acylhydrazides of formula I or compositions comprising them can additionally be employed in a further number of crop plants for eliminating undesirable plants. Examples of suitable crops are the following: Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, lpomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera and Zea mays.

Preferred crops are the following: Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays.

The acylhydrazides of formula I according to the invention can also be used in genetically modified plants. The term "genetically modified plants" is to be understood as plants, which genetic material has been modified by the use of recombinant DNA techniques in a way that under natural circumstances it cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-transtional modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun® sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady^{®} (glyphosate tolerant, Monsanto, USA), Cultivance® (imidazolinone tolerant, BASF SE, Germany) and LibertyLink^{®} (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as ä-endotoxins, e. g. CryIA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 orVIP3A; insecticidal proteins of bacteria colonizing nematodes, e. g. Photorhabdus spp. or Xenorhabdus spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be under-stood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are dis-closed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars produ-cing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to in-crease the resistance or tolerance of those plants to bacterial, viral or fungal pathogens. Examples of such proteins are the so-called "pathogenesis-related proteins" (PR proteins, see, e.g. EP-A 392 225), plant disease resistance genes (e. g. potato culti-vars, which express resistance genes acting against Phytophthora infestans derived from the mexican wild potato Solanum bulbocastanum) or T4-lyso-zym (e.g. potato cultivars capable of synthesizing these proteins with increased resistance against bacteria such as Erwinia amylvora). The methods for producing such genetically modi-fied plants are generally known to the person skilled in the art and are described, e.g. in the publications mentioned above.

Furthermore, plants are also covered that are by the use of recombinant DNA techniques capable to synthesize one or more proteins to increase the productivity (e.g. bio mass production, grain yield, starch content, oil content or protein content), tolerance to drought, salinity or other growth-limiting environ-mental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce healthpromoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e.g. potatoes that produce increased amounts of amylopectin (e.g. Amflora^{®} potato, BASF SE, Germany).

The acylhydrazides of formula I, or the herbicidal compositions comprising the acylhydrazides of formula I, can be used, for example, in the form of ready-to-spray aqueous solutions, powders, suspensions, also highly concentrated aqueous, oily or other suspensions or dispersions, emulsions, oil dispersions, pastes, dusts, materials for broadcasting, or granules, by means of spraying, atomizing, dusting, spreading, watering or treatment of the seed or mixing with the seed. The use forms depend on the intended purpose; in any case, they should ensure the finest possible distribution of the active ingredients according to the invention.

The herbicidal compositions comprise an herbicidal effective amount of at least one acylhydrazides of the formula I and auxiliaries which are customary for the formulation of crop protection agents.

Examples of auxiliaries customary for the formulation of crop protection agents are inert auxiliaries, solid carriers, surfactants (such as dispersants, protective colloids, emulsifiers, wetting agents and tackifiers), organic and inorganic thickeners, bactericides, antifreeze agents, antifoams, optionally colorants and, for seed formulations, adhesives.

The person skilled in the art is sufficiently familiar with the recipes for such formulations.

Examples of thickeners (i.e. compounds which impart to the formulation modified flow properties, i.e. high viscosity in the state of rest and low viscosity in motion) are polysaccharides, such as xanthan gum (Kelzan® from Kelco), Rhodopol® 23 (Rhone Poulenc) or Veegum® (from R.T. Vanderbilt), and also organic and inorganic sheet minerals, such as Attaclay® (from Engelhardt).

Examples of antifoams are silicone emulsions (such as, for example, Silikon^{®} SRE, Wacker or Rhodorsil® from Rhodia), long-chain alcohols, fatty acids, salts of fatty acids, organofluorine compounds and mixtures thereof.

Bactericides can be added for stabilizing the aqueous herbicidal formulations. Examples of bactericides are bactericides based on diclorophen and benzyl alcohol hemiformal (Proxel® from ICI or Acticide® RS from Thor Chemie and Kathon® MK from Rohm & Haas), and also isothiazolinone derivates, such as alkylisothiazolinones and benzisothiazolinones (Acticide MBS from Thor Chemie).

Examples of antifreeze agents are ethylene glycol, propylene glycol, urea or glycerol.

Examples of colorants are both sparingly water-soluble pigments and water-soluble dyes. Examples which may be mentioned are the dyes known under the names Rhodamin B, C.I. Pigment Red 112 and C.I. Solvent Red 1, and also pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Examples of adhesives are polyvinylpyrrolidone, polyvinyl acetate, polyvinyl alcohol and tylose.

Suitable inert auxiliaries are, for example, the following: mineral oil fractions of medium to high boiling point, such as kerosene and diesel oil; furthermore coal tar oils and oils of vegetable or animal origin, aliphatic, cyclic and aromatic hydrocarbons, for example paraffin, tetrahydronaphthalene, alkylated naphthalenes and their derivatives, alkylated benzenes and their derivatives, alcohols such as methanol, ethanol, propanol, butanol and cyclohexanol, ketones such as cyclohexanone or strongly polar solvents, for example amines such as N-methylpyrrolidone, and water.

Suitable carriers include liquid and solid carriers. Liquid carriers include e.g. non-aqeuos solvents such as cyclic and aromatic hydrocarbons, e.g. paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, e.g. amines such as N-methylpyrrolidone, and water as well as mixtures thereof. Solid carriers include e.g. mineral earths such as silicas, silica gels, silicates, talc, kaolin, limestone, lime, chalk, bole, loess, clay, dolomite, diatomaceous earth, calcium sulfate, magnesium sulfate and magnesium oxide, ground synthetic materials, fertilizers such as ammonium sulfate, ammonium phosphate, ammonium nitrate and ureas, and products of vegetable origin, such as cereal meal, tree bark meal, wood meal and nutshell meal, cellulose powders, or other solid carriers.

Suitable surfactants (adjuvants, wetting agents, tackifiers, dispersants and also emulsifiers) are the alkali metal salts, alkaline earth metal salts and ammonium salts of aromatic sulfonic acids, for example lignosulfonic acids (e.g. Borrespers-types, Borregaard), phenolsulfonic acids, naphthalenesulfonic acids (Morwet types, Akzo Nobel) and dibutylnaphthalenesulfonic acid (Nekal types, BASF AG), and of fatty acids, alkyl- and alkylarylsulfonates, alkyl sulfates, lauryl ether sulfates and fatty alcohol sulfates, and salts of sulfated hexa-, hepta- and octadecanols, and also of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl or tributylphenyl polyglycol ether, alkylaryl polyether alcohols, isotridecyl alcohol, fatty alcohol/ethylene oxide condensates, ethoxylated castor oil, polyoxyethylene alkyl ethers or polyoxypropylene alkyl ethers, lauryl alcohol polyglycol ether acetate, sorbitol esters, lignosulfite waste liquors and proteins, denaturated proteins, polysaccharides (e.g. methylcellulose), hydrophobically modified starches, polyvinyl alcohol (Mowiol types Clariant), polycarboxylates (BASF AG, Sokalan types), polyalkoxylates, polyvinylamine (BASF AG, Lupamine types), polyethyleneimine (BASF AG, Lupasol types), polyvinylpyrrolidone and copolymers thereof.

Powders, materials for broadcasting and dusts can be prepared by mixing or concomitant grinding the active ingredients together with a solid carrier.

Granules, for example coated granules, impregnated granules and homogeneous granules, can be prepared by binding the active ingredients to solid carriers.

Aqueous use forms can be prepared from emulsion concentrates, suspensions, pastes, wettable powders or water-dispersible granules by adding water.

To prepare emulsions, pastes or oil dispersions, the acylhydrazides of the formula I, either as such or dissolved in an oil or solvent, can be homogenized in water by means of a wetting agent, tackifier, dispersant or emulsifier. Alternatively, it is also possible to prepare concentrates comprising active compound, wetting agent, tackifier, dispersant or emulsifier and, if desired, solvent or oil, which are suitable for dilution with water.

The concentrations of the acylhydrazides of the formula I in the ready-to-use preparations (formulations) can be varied within wide ranges. In general, the formulations comprise approximately from 0.001 to 98% by weight, preferably 0.01 to 95% by weight of at least one active ingredient. The active ingredients are employed in a purity of from 90% to 100%, preferably 95% to 100% (according to NMR spectrum).

In the formulation of the acylhydrazides of formula I according to the present invention the active ingredients, e.g. the acylhydrazides of formula I, are present in suspended, emulsified or dissolved form. The formulation according to the invention can be in the form of aqueous solutions, powders, suspensions, also highly-concentrated aqueous, oily or other suspensions or dispersions, aqueous emulsions, aqueous microemulsions, aqueous suspo-emulsions, oil dispersions, pastes, dusts, materials for spreading or granules.

The acylhydrazides of formula I according to the present invention can, for example, be formulated as follows:
1. Products for dilution with water
   A Water-soluble concentrates 10 parts by weight of active compound are dissolved in 90 parts by weight of water or a water-soluble solvent. As an alternative, wetters or other adjuvants are added. The active compound dissolves upon dilution with water. This gives a formulation with an active compound content of 10% by weight.
   B Dispersible concentrates 20 parts by weight of active compound are dissolved in 70 parts by weight of cyclohexanone with addition of 10 parts by weight of a dispersant, for example polyvinylpyrrolidone. Dilution with water gives a dispersion. The active compound content is 20% by weight.
   C Emulsifiable concentrates 15 parts by weight of active compound are dissolved in 75 parts by weight of an organic solvent (eg. alkylaromatics) with addition of calcium dodecylbenzenesulfonate and castor oil ethoxylate (in each case 5 parts by weight). Dilution with water gives an emulsion. The formulation has an active compound content of 15% by weight.
   D Emulsions 25 parts by weight of active compound are dissolved in 35 parts by weight of an organic solvent (eg. alkylaromatics) with addition of calcium dodecylbenzenesulfonate and castor oil ethoxylate (in each case 5 parts by weight). This mixture is introduced into 30 parts by weight of water by means of an emulsifier (Ultraturrax) and made into a homogeneous emulsion. Dilution with water gives an emulsion. The formulation has an active compound content of 25% by weight.
   E Suspensions In an agitated ball mill, 20 parts by weight of active compound are comminuted with addition of 10 parts by weight of dispersants and wetters and 70 parts by weight of water or an organic solvent to give a fine active compound suspension. Dilution with water gives a stable suspension of the active compound. The active compound content in the formulation is 20% by weight.
   F Water-dispersible granules and water-soluble granules 50 parts by weight of active compound are ground finely with addition of 50 parts by weight of dispersants and wetters and made into water-dispersible or water-soluble granules by means of technical appliances (for example extrusion, spray tower, fluidized bed). Dilution with water gives a stable dispersion or solution of the active compound. The formulation has an active compound content of 50% by weight.
   G Water-dispersible powders and water-soluble powders 75 parts by weight of active compound are ground in a rotor-stator mill with addition of 25 parts by weight of dispersants, wetters and silica gel. Dilution with water gives a stable dispersion or solution of the active compound. The active compound content of the formulation is 75% by weight.
   H Gel formulations In a ball mill, 20 parts by weight of active compound, 10 parts by weight of dispersant, 1 part by weight of gelling agent and 70 parts by weight of water or of an organic solvent are mixed to give a fine suspension. Dilution with water gives a stable suspension with active compound content of 20% by weight.
2. Products to be applied undiluted
   I Dusts 5 parts by weight of active compound are ground finely and mixed intimately with 95 parts by weight of finely divided kaolin. This gives a dusting powder with an active compound content of 5% by weight.
   J Granules (GR, FG, GG, MG) 0.5 parts by weight of active compound are ground finely and associated with 99.5 parts by weight of carriers. Current methods here are extrusion, spray-drying or the fluidized bed. This gives granules to be applied undiluted with an active compound content of 0.5% by weight.
   K ULV solutions (UL) 10 parts by weight of active compound are dissolved in 90 parts by weight of an organic solvent, for example xylene. This gives a product to be applied undiluted with an active compound content of 10% by weight.

Aqueous use forms can be prepared from emulsion concentrates, suspensions, pastes, wettable powders or water-dispersible granules by adding water.

Application can be done before, during and/or after, preferably during and/or after, the emergence of the undesirable plants.

The acylhydrazides of the formula I or the herbicidal compositions comprising them can be applied pre-, post-emergence or pre-plant, or together with the seed of a crop plant. It is also possible to apply the herbicidal composition or active compounds by applying seed, pretreated with the herbicidal compositions or active compounds, of a crop plant. If the active ingredients are less well tolerated by certain crop plants, application techniques may be used in which the herbicidal compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the acylhydrazides of the formula I or the herbicidal compositions can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the acylhydrazides of the formula I according to the invention or the compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

The rates of application of the active acylhydrazide of formula I according to the present invention (total amount of acylhydrazide of formula I) are from 0,1 g/ha to 3000 g/ha, preferably 10 g/ha to 1000 g/ha of active substance (a.s.), depending on the control target, the season, the target plants and the growth stage.

In another preferred embodiment of the invention, the application rates of the acylhydrazides of formula I are in the range from 0.1 g/ha to 5000 g/ha and preferably in the range from 1 g/ha to 2500 g/ha or from 5 g/ha to 2000 g/ha of active substance (a.s.).

In another preferred embodiment of the invention, the application rate of the acylhydrazide of formula I is 0.1 to 1000 g/ha, preferably1 to 750 g/ha, more preferably 5 to 500 g/ha, of active substance.

To treat the seed, the acylhydrazides of formula I are generally employed in amounts of from 0.001 to 10 kg per 100 kg of seed.

To widen the spectrum of action and to achieve synergistic effects, the acylhydrazides of the formula I may be mixed with a large number of representatives of other herbicidal or growth-regulating active ingredient groups and then applied concomitantly. Suitable components for mixtures are, for example, 1,2,4-thiadiazoles, 1,3,4-thiadiazoles, amides, aminophosphoric acid and its derivatives, aminotriazoles, anilides, (het)aryloxyalkanoic acids and their derivatives, benzoic acid and its derivatives, benzothiadiazinones, 2-aroyl-1,3-cyclohexanediones, 2-hetaroyl-1,3-cyclohexanediones, hetaryl aryl ketones, benzylisoxazolidinones, meta-CF₃-phenyl derivatives, carbamates, quinolinecarboxylic acid and its derivatives, chloroacetanilides, cyclohexenone oxime ether derivatives, diazines, dichloropropionic acid and its derivatives, dihydrobenzofurans, dihydrofuran-3-ones, dinitroanilines, dinitrophenols, diphenyl ethers, dipyridyls, halocarboxylic acids and their derivatives, ureas, 3-phenyluracils, imidazoles, imidazolinones, N-phenyl-3,4,5,6-tetrahydrophthalimides, oxadiazoles, oxiranes, phenols, aryloxy- and hetaryloxyphenoxypropionic esters, phenylacetic acid and its derivatives, 2-phenylpropionic acid and its derivatives, pyrazoles, phenylpyrazoles, pyridazines, pyridinecarboxylic acid and its derivatives, pyrimidyl ethers, sulfonamides, sulfonylureas, triazines, triazinones, triazolinones, triazolecarboxamides, uracils, phenyl pyrazolines and isoxazolines and derivatives thereof.

It may furthermore be beneficial to apply the acylhydrazides of the formula I alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

Moreover, it may be useful to apply the acylhydrazides of the formula I in combination with safeners. Safeners are chemical compounds which prevent or reduce damage on useful plants without having a major impact on the herbicidal action of the ... of the formula I towards unwanted plants. They can be applied either before sowings (e.g. on seed treatments, shoots or seedlings) or in the pre-emergence application or post-emergence application of the useful plant. The safeners and the acylhdrazides of the formula I can be applied simultaneously or in succession.

Suitable safeners are e.g. (quinolin-8-oxy)acetic acids, 1-phenyl-5-haloalkyl-1 H-1,2,4-triazol-3-carboxylic acids, 1-phenyl-4,5-dihydro-5-alkyl-1 H-pyrazol-3,5-dicarboxylic acids, 4,5-dihydro-5,5-diaryl-3-isoxazol carboxylic acids, dichloroacetamides, alphaoximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

The acylhydrazides of formula I according to the present invention can be used as pharmaceutical active ingredients (medicaments), especially for treating or preventing parasitic and/or bacterial infections in a subject.

The term "subject", as used in connection with the method of treating or preventing parasitic and/or bacterial infections refers to a living animal, e.g. mammals and birds, preferably mammals, most preferably humans; also preferably birds, also most preferably poultry.

Parasitic and bacterial infections can occur in both, humans and animals.

Examples for parasitic infections are malaria, coccidiosis, toxoplasmosis, leishmaniasis and trypanosomiasis (e.g. African Sleeping Sickness, South American Chagas Disease). These parasitic infections are caused by parasitic Protozoa, e.g. of the genus Plasmodium (e.g. the species P. falciparum, P. maliariae, P. ovale and P. vivax), Eimeria (e.g. the species E. tenella and E. necatrix), Toxoplasma (e.g. the species T. gondii), Leishmania (e.g. the species L. donovani) and Trypanosoma (e.g the species T. brucei and T. cruzei). Plasmodium belongs to the order of Haemosprida, whereas Eimeria and Toxoplasma belong both to the order of Eucoccidiorida. Haemosprida and Eucoccidiorida both belong to the phylum Apicomplexa. Leishmania and Trypanosoma belong both to the order of Trypanosomatida, which itself belongs to the phylum Euglenozoa. Aplicomplexa and Euglenozoa are both examples for "parasitic Protozoa".

Examples for bacterial infections are tuberculosis, leprosy, mycetoma, listeriosis, meningitis, botulism, tetanus, trachoma, urethritis, pelvic inflammatoric disease, typhoid fever, paratyphoid fever and foodborne illness slmonellosis. These infections are caused by bacteria, e.g. of the genus Mycobacterium (e.g the species M. leprae, M. tuberculosis, M. ulceran), Streptomyces (e.g. the species S. sudanensis, S. somaliensis), Listeria (e.g. the species L. monocytogenes), Clostridium (e.g. the species C. botulinum, C. difficile, C. perfringens, C. tetani), Chlamydia (e.g. the species C. pneumoniae, C. trachomatis) and Salmonella (e.g. the species S. bongori, S. enterica containing the sub-subspecies S. typhi and S. typhimurium). Mycobacterium and Streptomyces both belong to the order of Actinomycetales, whereas Listeria belongs to the order of Bacillaes, Clostridium belongs to the order of Clostridiales, Chlamydia belongs to the order of Chlamydiales and Salmonella belongs to the order of Enterobacteriales. Actinomycetales of the phylum Actinobacteria as well as Bacillaes and Clostridiales, both of the phylum Firmicutes, are examples for Gram-positive bacteria. Chlamydiales (phylum Clamydiae) and Enterobacteriales (phylum Gamma Probacteria) are both examples for Gram-negative bacteria.

In one embodiment of the invention, the acylhydrazides of formula I are used as pharmaceutical active ingredients for treating or preventing parasitic and/or bacterial infections caused by parasitic Protozoa and/or Gram-positive bacteria.

In another embodiment of the invention, the acylhydrazides of formula I are used as pharmaceutical active ingredients for treating or preventing parasitic infections caused by parasitic Protozoa,

preferably caused by Apicomplexa and Englenozoa,

more preferably caused by Haemasporida, Eucoccidiorida and Trypanosomatida, particularly preferred caused by Plasmodium, Eimeria, Toxoplasma, Leishmania and Trypanosoma, especially preferred caused by Plasmodium, most preferably caused by P. falciparum and P. malariae.

In another embodiment of the invention, the acylhydrazides of formula I are used as pharmaceutical active ingredients for treating or preventing parasitic infections caused by Apicomplexa, preferably caused by Haemasporida and Eucoccidiorida, more preferably caused by Plasmodium, Eimeria and Toxoplasma, particularly preferred caused by P. falciparum, P. malariae, E. tenellae and T. gondii.

In another embodiment of the invention, the acylhydrazides of formula I are used as pharmaceutical active ingredients for treating or preventing parasitic infections caused by Englenozoa, more preferably caused by Trypanosomatida, particularly preferred caused by Leishmania and Trypanosoma, especially preferred caused by L. donovani, T. brucei and T. cruzei.

In another embodiment of the invention, the acylhydrazides of formula I are used as pharmaceutical active ingredients for treating or preventing bacterial infections caused by Actinobacteria, Firmicutes, Chlamydiae and Gamma Probacteria, preferably caused by Actinomycetales, Bacillaes, Clostridiales, Chlamydiales and Enterocbacteriales, more preferably caused by Mycobacterium, Streptomyces, Listeria, Clostridium, Chlamydia and Salmonella particularly preferred caused by Mycobacterium, Streptomyces, Listeria and Clostridium, especially preferred caused by Mycobacterium, Listeria and Clostridium.

In another embodiment of the invention, the acylhydrazides of formula I are used as pharmaceutical active ingredients for treating or preventing bacterial infections caused by Gram-positive bacteria, preferably caused by Actinobacteria and Firmicutes, more preferably caused by Actinomycetales, Bacillaes and Clostridiales, particularly preferred caused by Mycobacterium, Streptomyces, Listeria and Clostridium, especially preferred caused by M. leprae, M. tuberculosis, L. monocytogenes, C. botulinum and C. tetani; most preferably caused by M. leprae, M. tuberculosis, C. botulinum and C. tetani.

In another embodiment of the invention, the acylhydrazides of formula I are used as pharmaceutical active ingredients for treating or preventing bacterial infections caused by Gram-negative bacteria, preferably caused by Chlamydiales and Enterobacteriales, more preferably caused by Chlamydia and Salmonella, particularly preferred caused by C. pneumoniae, C. trachomatis, S. bongori and S. enterica.

The term "pharmaceutically acceptable", as used in connection with the compounds and compositions of the invention, refers to molecular entities and other ingredients of such compositions that are physiologically tolerable and do not typically produce untoward reactions when administered to a subject, preferably to a mammal (e.g. a human). Preferably, the term "pharmaceutically acceptable" means approved by a regulatory agency or listed in e.g. the U.S. Pharmacopeia for use in mammals and particularly in humans.

Compounds of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt is not critical, provided that it is non-toxic and does not substantially interfere with the desired pharmacological activity.

The term "therapeutically effective" applied to dose or amount refers to that quantity of a compound or pharmaceutical composition that is sufficient to result in a desired activity upon administration to a living animal body in need thereof.

For therapeutic use, salts of the acylhadrazides of formula I are those wherein the counter-ion is pharmaceutically acceptable. However, salts of acids and bases, which are non-pharmaceutically acceptable, may also find use, for example, in the preparation and purification of pharmaceutically acceptable compounds.

All salts whether pharmaceutically acceptable or not are included within the ambit of the present invention. The pharmaceutically acceptable salts as mentioned above are meant to comprise the therapeutically active non-toxic salt forms, which the compounds of formula I are able to form. The latter can conveniently be obtained by treating the base form with such appropriate acids as inorganic acids, e.g. hydrohalic acids such as hydrochloric, hydrobromic and the like; sulfuric acid; nitric acid; phosphoric acid and the like; or organic acids such as acetic, propanoic, hydroxyacetic, 2-hydroxypropanoic, oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfonic, 2-hydroxybenzoic, 4-amino-2-hydroxybenzoic and the like acids. Conversely, the salt form can be converted by treatment with alkali into the free base form.

The active ingredients of formula I of the invention, together with at least one auxiliary customary for formulating pharmaceutical, e.g. one or more conventional adjuvants, carriers, or diluents, may be placed into the form of pharmaceutical compositions and unit dosages thereof.

In such form the active ingredients of formula I may be employed as solid, such as coated or uncoated tablets or filled capsules, or liquid, such as solutions, suspensions, emulsions, elixirs, or capsules filled with the same, all for oral use; in the form of suppositories or capsules for rectal administration or in the form of sterile injectable solutions for parenteral, including intravenous or subcutaneous, use.

Such pharmaceutical compositions and unit dosage forms thereof may comprise conventional or new ingredients in conventional or special proportions, with or without additional active compounds or principles, and such unit dosage forms may contain any suitable effective amount of the active ingredient commensurate with the intended daily dosage range to be employed. Tablets containing from 0.1 to 600 milligrams of active ingredient, more preferred from 0.5 to 500 milligrams per tablet, are suitable representative unit dosage forms.

The term "carrier" applied to pharmaceutical compositions of the invention refers to a diluent, excipient or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. A. R. Gennaro, 20th Edition, describes suitable pharmaceutical carriers in "Remington: The Science and Practice of Pharmacy".

Due to their high degree of activity, the high metabolic stability and their low toxicity, together presenting favorable therapeutic index, the active compounds of formula I of the invention may be administered to a subject, e.g., a living animal (including a human) body in need thereof, for the treatment, alleviation, or amelioration, palliation, or elimination of an indication or condition which is susceptible thereto, or representatively of an indication or condition set forth elsewhere in this application, preferably concurrently, simultaneously, or together with one or more pharmaceutically-acceptable excipients, carriers or diluents.

Suitable dosage ranges are 0.1 to 1000 milligrams daily, preferably 1 to 500 milligrams daily, and especially 5 to 500 milligrams daily, depending as usual upon the exact mode of administration, form in which administered, the indication toward which the administration is directed, the subject involved and the body weight of the subject involved, and the preference and experience of the physician or veterinarian in charge.

The acylhydrazides of formula I can be applied in the form of a pharmaceutical composition whether by oral, rectal, or parental (including intravenous and subcutaneous) or in some cases even topical route, in an effective amount.

The active compound of formula I of the present invention may be administered orally, topically, parenterally, or mucosally (e.g., buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like (see Remington: The Science and Practice of Pharmacy, 20th Edition). The orally administered medicaments may be administered in the form of a time-controlled release vehicle, including diffusion-controlled systems, osmotic devices, dissolution-controlled matrices, and erodible/degradable matrices.

For oral administration in the form of a tablet or capsule, the active component of formula I may be combined with a non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the drug components may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms.

The tablets containing as active compound a compound of formula I may be coated by methods well known in the art. The compositions of the invention containing as active compound a compound of formula I may be also introduced in beads, microspheres or microcapsules, e.g., fabricated from polyglycolic acid/lactic acid (PGLA). Liquid preparations for oral administration may take the form of, for example, solutions, syrups, emulsions or suspensions, or they may be presented as a dry product for reconstitution with water or other suitable vehicle before use. Preparations for oral administration may be suitably formulated to give controlled or postponed release of the active compound.

The active drugs of formula I may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known. Drugs of the invention containing as active compound a compound of formula I may also be delivered by the use of monoclonal antibodies as individual carriers to which the compound molecules are coupled. Active drugs may also be coupled with soluble polymers as targetable drug carriers. Such polymers include polyvinyl-pyrrolidone, pyran copolymer, polyhydroxy-propyl methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention containing as active compound a compound of formula I may be conveniently delivered in the form of an aerosol spray presentation from pressurized packs or a nebulizer, with the use of a suitable propellant, e.g., dichlorodifluoromethane, trichlorofluoromethane, dichlorotetra-fluoroethane, carbon dioxide, or other suitable gas. In the case of a pressurized aerosol, the dosage unit may be determined by providing a valve to deliver a metered amount. Capsules and cartridges of, e.g., gelatin for use in an inhaler or insufflator can be formulated containing a powder mix of the compound and a suitable powder base such as lactose or starch.

The formulations of the invention containing a compound of formula I may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, e.g., in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient can be in powder form for reconstitution with a suitable vehicle, e.g., sterile pyrogen-free water, before use.

Compositions of the present invention containing a compound of formula I may also be formulated for rectal administration, e.g., as suppositories or retention enemas (e.g., containing conventional suppository bases such as cocoa butter or other glycerides).

The compositions containing a compound of formula I may, if desired, be presented in a pack or dispenser device, which may contain one or more unit dosage forms containing the active ingredient and/or may contain different dosage levels to facilitate dosage titration. The pack may, for example, comprise metal or plastic foil, such as a blister pack.

Compositions of the invention formulated in a compatible pharmaceutical carrier may also be prepared, placed in an appropriate container, and labeled for treatment of an indicated condition.

As disclosed herein, the dose of the components in the compositions of the present invention is determined to ensure that the dose administered continuously or intermittently will not exceed an amount determined after consideration of the results in test animals and the individual conditions of a patient.

A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, e.g., by determining the LD50 (the dose lethal to 50% of the population) and the ED50 (the dose therapeutically effective in 50% of the population). The dose ratio between therapeutic and toxic effects is the therapeutic index and it may be expressed as the ratio ED50/LD50. Compositions that exhibit large therapeutic indices are preferred.

Hereinbelow, the preparation of the acylhydrazides of the formula I is illustrated by examples; however, the subject matter of the present invention is not limited to the examples given.
1) Pyridine-2-carboxylic acid [7-amino-pyrrolo[3,4-b]pyrazin-(5Z)-ylidene]-hydrazide 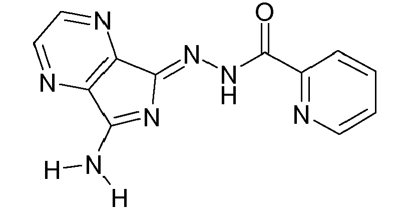 114 mg (0.88 mmol) Pyrazin-2,3-dicarbonitril and 110 mg (0.80 mmol) Pyridine-2-carboxylic acid hydrazide were suspended in ethanol (EtOH), and the suspension was heated to reflux for 20 min. The resulting solution was cooled to 50°C, 160 µl (0.080 mmol) 0.5 M NaOCH₃ in methanol (MeOH) was added and the solution was heated to reflux for 8 h. After cooling to room temperature (RT) 4 ml diethylether (Et₂O) were added, the reddish precipitate was filtered, washed with Et₂O and dried in vacuum to yield 119 mg of the title compound in form of a red brown powder.
   Mp: 261-264°C; MS: M+H = 268
   1 H-NMR (DMSO): 11.9 (br, 1 H); 8.8 (s, 1 H); 8.9 (s, 1 H); 8.7 (s, 1 H); 8.6 (br, 2H); 8.2 (s, 1 H); 8.1 (s, 1H);7.7(s, 1 H)
2) 4-Dimethylamino-pyridine-2-carboxylic acid [1-amino-benzo[e]isoindol-(3Z)-ylidene]-hydrazide 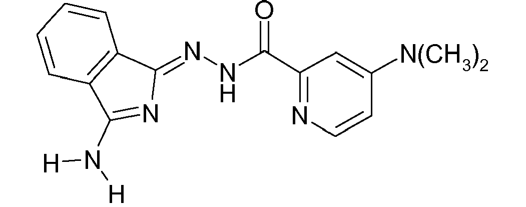 118 mg (0.66 mmol) Naphthalene-1,2-dicarbonitrile and 108 mg (0.60 mmol) 4-Dimethylamino-pyridine-2-carboxylic acid hydrazide were suspended in EtOH and the suspension was heated to reflux for 20 min. The resulting solution was cooled to 50°C, 120 µl (0.060 mmol) 0.5M NaOCH₃ in MeOH was added and the solution was heated to reflux for 8 h. After cooling to RT 3ml Et₂O were added, the brownish precipitate was filtered, washed with Et₂O and dried in vacuum to yield 215 mg of the title compound in form of a brown powder.
   MS: M+H = 369; 1:2 mixture of regioisomers
   1H-NMR (DMSO, major isomer): 12.0 (br, 1H);9.1 (d, 1 H); 8.4 (br, 2H); 8.3 (s, 1 H); 8.1 (m, 2H); 7.9 (d, 1 H); 7.6-7.8 (m, 2H); 7.4 (s, 1 H); 6.9 (s, 1 H); 3.2 (s, 6H)
3) 4-Morpholin-4-yl-pyridine-2-carboxylic acid [3-amino-isoindol-(1Z)-ylidene]-hydrazide 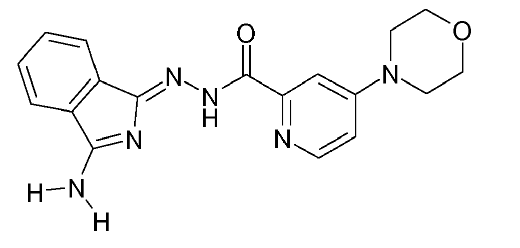 112 mg (0.77 mmol) 3-Imino-3H-isoindol-1-ylamine and 156 mg (0.70 mmol) 4-Morpholin-4-yl-pyridine-2-carboxylic acid hydrazide were suspended in EtOH and the suspension was heated to reflux 4h. After cooling to RT 3.5 ml Et₂O were added, the yellow precipitate was filtered, washed with Et₂O and dried in vacuum to yield 233 mg of the title compound in form of a yellow powder.
   Mp: 300-301 °C; MS: M+H = 351
   HRMS (MALDI; M+Na): 373.1388
   1 H-NMR (DMSO): 12.0 (s, 1 H); 8.9 (br, 1 H); 8.7 (br, 1 H); 8.4 (s, 1 H); 8.0 (d, 1 H); 7.9 (d, 1 H); 7.7 (m, 3H); 7.1 (s, 1 H); 3.8 (s, 4H); 3.5 (s, 4H)
4) N-{3-[(Pyridine-2-carbonyl)-hydrazono]-3H-isoindol-1-yl}-acetamide 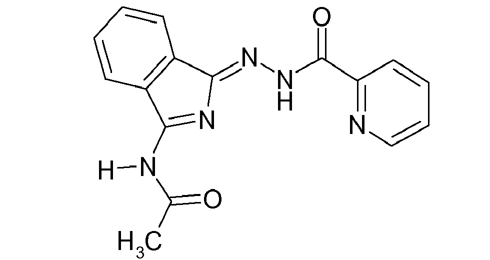 53 mg (0.20 mmol) Pyridine-2-carboxylic acid [3-amino-isoindol-(1Z)-ylidene]-hydrazide known from Eur. J. of Org. Chem. (12) 2006, 2833-2842 were dissolved in 4 ml acetic anhydride (AC₂O) and refluxed for 1 h. After cooling the yellow precipitate was filtrated and dried in vacuum to yield 52 mg of the title compound in form of a yellow powder.
   MS: M+H = 308; HRMS (MALDI; +Na): 330.0959
   1 H-NMR (DMSO): 12.3 (s, 1 H); 11.6 (s, 1 H); 8.8 (s, 1 H); 8.3 (m, 2H); 8.2 (m, 1 H); 8.0 (s, 1 H); 7.6-7.8 (m, 3H); 2.5 (s, 3H)
5) 3-(2-(dimethylamino)ethylthio)phthalonitril 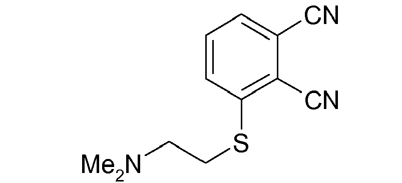 692 mg (4.0 mmol) 3-Nitrophthalonitril, 623 mg (4.4 mmol) 2-(dimethylamino)-ethanethiol hydrochloride and 1399 mg (13.2 mmol) were suspended in DMF and stirred at RT for 2h. The suspension was added to a mixture of 20 ml brine and 20 ml water, the colorless crystals were filtrated, washed with water and dried in vacuum to yield 820 mg of the title compound in form of a colorless powder. MS: M+H = 232
   1 H-NMR (CDCl3): 7.5-7.7 (m, 3H); 3.1 (d, 2H); 2.6 (m, 2H); 2.2 (m, 6H)
6) Isoquinoline-3-carboxylic acid [3-amino-4-(2-dimethylamino-ethylsulfanyl)-isoindol-(1 Z)-ylidene]-hydrazide 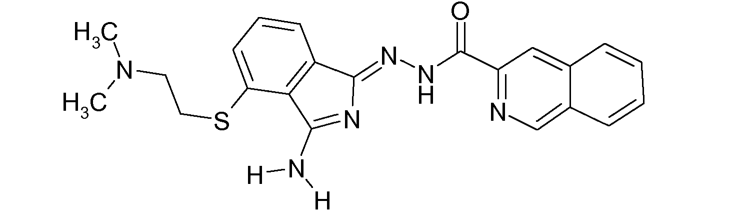 102 mg (0.44 mmol) 3-(2-(dimethylamino)ethylthio)phthalonitril and 75 mg (0.40 mmol) Isoquinoline-3-carboxylic acid hydrazide were suspended in EtOH and the suspension was heated to reflux for 20 min. The resulting solution was cooled to 50°C, 80 µl (0.040 mmol) 0.5 M NaOMe in MeOH was added and the solution was heated to reflux for 4 h. After cooling to RT 2 ml Et₂O were added, the yellow precipitate was filtered, washed with Et₂O and dried in vacuum to yield 30 mg of the title compound in form of a yellow powder.
   Mp: 269-271°C; MS: M+H = 419; HRMS (MALDI, M+Na): 441.1460
   1 H-NMR (DMSO): 12.1 (s, 1 H); 9.4 (s, 1 H); 8.7 (s, 1 H); 8.5 (br, 2H); 8.3 (m, 2H); 7.9 (m, 2H); 7.7 (s, 1 H); 7.5 (m, 2H); 3.2 (m, 2H); 2.6 (m, 2H); 2.2 (s, 6H)
7) 4-Methoxy-pyridine-2-carboxylic acid [3-amino-isoindol-(1Z)-ylidene]-hydrazide 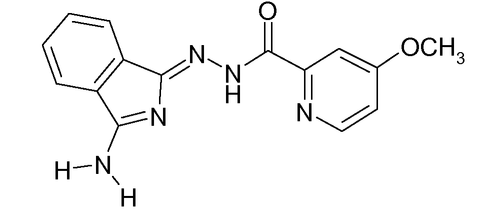 174 mg (1.2 mmol) 3-Imino-3H-isoindol-1-ylamine and 181 mg (1.1 mmol) 4-Methoxy-pyridine-2-carboxylic acid hydrazide were suspended in EtOH and the suspension was heated to reflux 8 h. After cooling to RT 3 ml Et₂O were added, the yellow precipitate was filtered, washed with Et₂O and dried in vacuum to yield 265 mg of the title compound in form of a yellow powder.
   Mp: 297-298°C; MS: M+H = 296; HRMS (MALDI; M+Na): 318.0969
   1 H-NMR (DMSO): 11.8 (s, 1 H); 8.8 (s, 1 H); 8.6 (s, 1 H); 8.5 (s, 1 H); 7.9 (d, 1 H); 7.8 (s, 1 H); 7.7 (s, 1 H); 7.5 (m, 2H); 7.2 (d, 1 H); 3.9 (s, 3H)
8) 4-Methoxy-pyridine-2-carboxylic acid [3-amino-isoindol-(1Z)-ylidene]-hydrazide hydrochloride 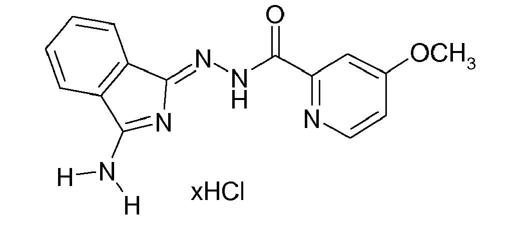 50 mg (0.17 mmol) 4-Methoxy-pyridine-2-carboxylic acid [3-amino-isoindol-(1Z)-ylidene]-hydrazide were suspended in 2ml 1M HCl/MeOH, evaporated on a Rotovap at 50°C and dried in vacuum to yield 57 mg of the title compound in form of a yellow powder.
   MS: M+H = 296
9) 1-Methyl-1H-imidazole-2-carboxylicacid [3-amino-isoindol-(1Z)-ylidene]-hydrazide 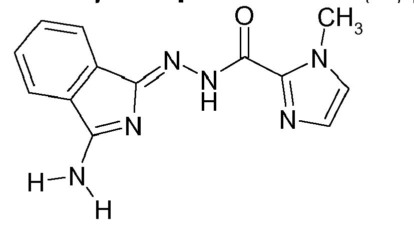 282 mg (2.2 mmol) Phthalonitril and 280 mg (2.0 mmol) 1-Methyl-1 H-imidazole-2-carboxylic acid hydrazide were suspended in EtOH and the suspension was heated to reflux for 20 min. The resulting solution was cooled to 50°C, 400 µl (0.20 mmol) 0.5 M NaOMe in MeOH was added and the solution was heated to reflux for 4 h. After cooling to RT 10 ml Et₂O were added, the yellow precipitate was filtered, washed with Et₂O and dried in vacuum to yield 345 mg of the title compound in form of a yellow powder.
   Mp: 278-280°C; MS: M+H = 269
   1 H-NMR (DMSO): 11.3 (br, 1 H); 8.6 (br, 2H); 7.9 (d, 1 H); 7.8 (s, 1 H); 7.6 (m, 2H); 7.4 (s, 1 H); 7.0 (s, 1 H); 4.0 (s, 3H)
10) Pyridine-2-carboxylic acid [3-amino-4-[4-(2,2-dimethyl-propionyl)-piperazin-1-yl]-isoindol-(1 Z)-ylidene]-hydrazide 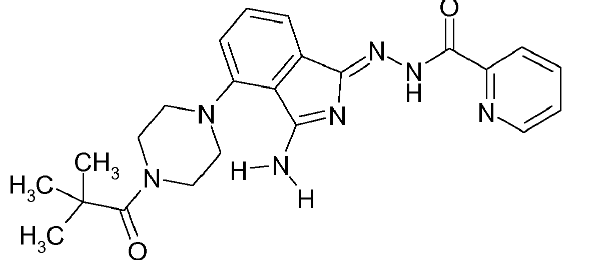 420 mg (1.98 mmol) 3-[4-(2,2-Dimethyl-propionyl)-piperazin-1-yl]-phthalonitrile and 247 mg (1.80 mmol) pyridine-2-carboxylic acid hydrazide were suspended in EtOH and the suspension was heated to reflux for 20 min. The resulting solution was cooled to 50°C, 360 µl (0.180 mmol) 0.5 M NaOMe in MeOH was added and the solution was heated to reflux for 8 h. After cooling to RT 9 ml Et₂O were added, the yellow precipitate was filtered, washed with Et₂O and dried in vacuum to yield 75 mg of the title compound in form of a yellow powder.
   Mp: 319-320°C; MS: M+H = 434; HRMS (MALDI, M+Na): 456.2126
   1 H-NMR (DMSO): 11.8 (s, 1 H); 9.0 (s, 1 H); 8.7 (s, 1 H); 8.2 (d, 1 H); 8.1 (t, 1 H); 7.7 (m, 1 H); 7.6 (m, 3H); 7.4 (s, 1 H); 3.6 (br, 4H); 3.0 (br, 4H); 1.2 (s, 9H)

Further 3-imino-3H-isoindol-1-ylamines of formula V, 3-hydrazono-3H-isoindol-1-ylamines of formula VI and acylhydrazides of formula I have been prepared inaccordance to the processes described above, and are, in addition to the compounds mentioned above, listed in tables 2 to 8 below. 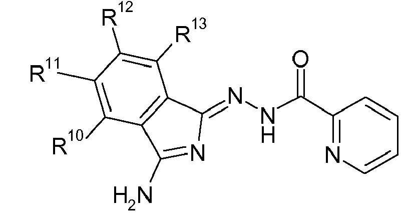 I with R¹ = A¹, where R⁵, R⁶, R⁷ and R⁸ are H; R²+R³ form B1; R⁴=H

**Table 2**

| No | R¹⁰ | R¹¹ | R¹² | R¹³ | Salt | LCMS (M+H) |
|---|---|---|---|---|---|---|
| 2.1. | H | H | H | H | -- | 266 |
| 2.2. | H | H | H | H | HCl | 266 |
| 2.3. | H | NO₂ | H | H | -- | 311 |
| 2.4. | H | C(CH₃)₃ | H | H | -- | 322 |
| 2.5. | H | SO₂C₆H₅ | H | H | -- | 406 |
| 2.6. | H | H | CH₃ | H | -- | 280 |
| 2.7. | H | H | O(n-C₆H₁₃) | H | -- | 366 |
| 2.8. | H | H | O-(n-C₁₂H₂₅) | H | -- | 451 |
| 2.9. | H | H | OCH₂CH₂-OCH₃ | H | -- | 340 |
| 2.10. | H | H | O(C₆H₄)O-(n-C₄H₉) | H | -- | 431 |
| 2.11. | H | H | NH₂ | H | -- | 281 |
| 2.12. | H | H | piperazinyl | H | -- | 350 |
| 2.13. | H | H | piperazinyl | H | -- | 400 |
| 2.14. | H | H | 4-hydroxy-piperinyl | H | -- | 365 |
| 2.15. | H | H | SC₆H₅ | H | -- | 374 |
| 2.16. | H | H | O(4-C(CH₃)₃-C₆H₄) | H | -- | 414 |
| 2.17. | H | H | H | OCH₂CHF₂ | -- | 346 |
| 2.18. | H | H | H | SCH₂CH₂-N(CH₃)₂ | -- | 370 |
| 2.19. | H | H | H | C₆H₅ | -- | 342 |
| 2.20. | H | H | H | O(2-F-C₆H₄) | -- | 376 |
| 2.21. | H | Cl | Cl | H | -- | 450 |
| 2.22. | H | CO₂CH₃ | CO₂CH₃ | H | -- | 382 |
| 2.23. | F | H | H | H | -- | 284 |
| 2.24. | F | H | H | H | -- | 314 |
| 2.25. | F | F | F | F | -- | 383 |
| 2.26. | Cl | H | H | H | -- | 301 |
| 2.27. | NO₂ | H | H | H | -- | 311 |
| 2.28. | NO₂ | H | H | H | HCl | 311 |
| 2.29. | OCH₃ | H | H | H | -- | 296 |
| 2.30. | OCH₃ | H | H | CH₃ | -- | 310 |
| 2.31. | OCH₂CHF₂ | H | H | H | -- | 346 |
| 2.32. | OCH₂CF₃ | H | H | H | -- | 364 |
| 2.33. | OCH₂CF₃ | H | H | H | HCl | 364 |
| 2.34. | OCH₂CH₂OH | H | H | H | -- | 326 |
| 2.35. | OCH₂CHCH₂ | H | H | H | -- | 322 |
| 2.36. | OCH₂CH₂O-CH₃ | H | H | H | -- | 340 |
| 2.37. | SCH₂CH₂OH | H | H | H | -- | 342 |
| 2.38. | SCH₂CH₂CH₂ OH | H | H | H | -- | 356 |
| 2.39. | SCH₂CH₂-N(CH₃)₂ | H | H | H | -- | 370 |
| 2.40. | SCH₂CH₂-N(CH₃)₂ | H | H | H | HCl | 370 |
| 2.41. | SCH₂CH₂-N(CH₂CH₃)₂ | H | H | H | -- | 398 |
| 2.42. | NHCH₂(C₆H₅) | H | H | H | -- | 371 |
| 2.43. | SO₂N(CH₃)₂ | H | H | H | -- | 373 |
| 2.44. | SO₂N(CH₃)₂ | H | H | H | HCl | 373 |
| 2.45. | SO₂N(C₂H₅)₂ | H | H | H | -- | 401 |
| 2.46. | morpholinyl | H | H | H | -- | 395 |
| 2.47. | N-morpholinyl | H | H | H | -- | 351 |
| 2.48. | NHCH₂CH₂-N-morpholinyl | H | H | H | -- | 515 |
| 2.49. | SCH₂CH₂-N-morpholinyl | H | H | H | -- | 412 |
| 2.50. | piperazinyl | H | H | H | -- | 350 |
| 2.51. | piperazinyl | H | H | H | -- | 407 |
| 2.52. | N-methyl-piperazinyl | H | H | H | -- | 364 |
| 2.53. | pivaloyl-piperazinyl | H | H | H | -- | 435 |
| 2.54. | N-allyl-piperazinyl | H | H | H | -- | 391 |
| 2.55. | NH-adamantyl | H | H | H | -- | 416 |
| 2.56. | C₆H₅ | H | H | H | -- | 342 |
| 2.57. | OC₆H₅ | H | H | H | -- | 358 |
| 2.58. | O(2-F-C₆H₄) | H | H | H | -- | 376 |
| 2.59. | O(4-F-C₆H₄) | H | H | H | -- | 376 |
| 2.60. | O(2-CH₃-C₆H₄) | H | H | H | -- | 372 |
| 2.61. | O(4-OCH₃-C₆H₄) | H | H | H | -- | 388 |
| 2.62. | SC₆H₅ | H | H | H | -- | 374 |
| 2.63. | SC₆H₅ | H | H | H | HCl | 374 |
| 2.64. | SO₂CH₂-CH₂OH | H | H | H | -- | 374 |
| 2.65. | S(4-Cl-C₆H₄) | H | H | H | -- | 409 |

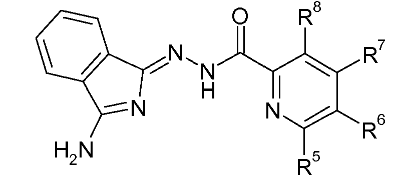

I with R¹ = A1; R² + R³ form B1, wherein R¹⁰, R¹¹, R¹² and R¹³ are H; R⁴ = H

**Table 3**

| No | R⁵ | R⁶ | R⁷ | R⁸ | Salt | LCMS (M+H) |
|---|---|---|---|---|---|---|
| 3.1. | CH₃ | H | H | H | -- | 280 |
| 3.2. | CF₃ | H | H | H | -- | 334 |
| 3.3. | OH | H | H | H | -- | 282 |
| 3.4. | OH | H | H | H | HCl | 282 |
| 3.5. | OCH₃ | H | H | H | -- | 296 |
| 3.6. | C₆H₅ | H | H | H | -- | 342 |
| 3.7. | H | CH₃ | H | H | -- | 280 |
| 3.8. | H | CH₂CH₂CH₂CH₃ | H | H | -- | 322 |
| 3.9. | H | Cl | H | H | -- | 300 |
| 3.10. | H | CN | H | H | -- | 291 |
| 3.11. | H | NO₂ | H | H | -- | 311 |
| 3.12. | H | N(C₂H₅)₂ | H | H | -- | 337 |
| 3.13. | H | H | Cl | H | -- | 300 |
| 3.14. | H | H | l | H | -- | 392 |
| 3.15. | H | H | CN | H | -- | 291 |
| 3.16. | H | H | OCH₃ | H | -- | 296 |
| 3.17. | H | H | OCH₃ | H | HCl | 296 |
| 3.18. | H | H | N(CH₃)₂ | H | -- | 309 |
| 3.19. | H | H | morpholinyl | H | -- | 351 |
| 3.20. | H | H | C₆H₅ | H | -- | 342 |
| 3.21. | H | H | H | OH | -- | 282 |
| 3.22. | -C₆H₄- | | H | H | -- | 316 |
| 3.23. | H | -C₆H₄- | | H | -- | 316 |
| 3.24. | H | OCH₃ | Cl | H | -- | 330 |

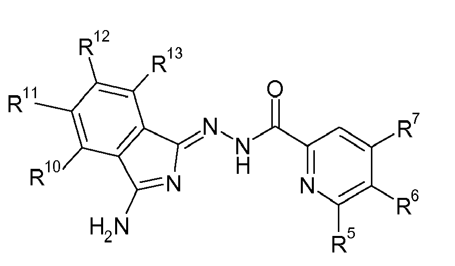

I with R¹ = A1 where R⁸ is H; R² + R³ form B1; R⁴ = H

**Table 4**

| No | R⁵ | R⁶ | R⁷ | R¹⁰ | R¹¹ | R¹² | R¹³ | LCMS (M+H) |
|---|---|---|---|---|---|---|---|---|
| 4.1 | H | CH₂CH₂-CH₂CH₃ | H | F | H | H | H | 322 |
| 4.2 | H | CH₂CH₂-CH₂CH₃ | H | H | H | H | SCH₂CH₂-N(CH₃)₂ | 426 |
| 4.3 | H | H | Cl | SCH₂CH₂-N(CH₃)₂ | H | H | H | 403 |
| 4.4 | H | NO₂ | H | H | Cl | Cl | H | 379 |
| 4.5 | H | H | Cl | F | F | F | F | 372 |
| 4.6 | H | -C₆H₄--C₆H₄- | | F | H | H | H | 334 |
| 4.7 | H | -C₆H₄--C₆H₄- | | H | H | H | SCH₂CH₂-N(CH₃)₂ | 420 |
| 4.8 | CF₃ | H | H | H | Cl | Cl | H | 402 |
| 4.9 | C₆H₅ | H | H | H | Cl | Cl | H | 410 |
| 4.10 | C₆H₅ | H | H | H | -C₆H₄- | | H | 392 |

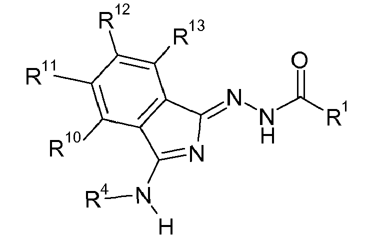

I with R² + R³ form B1

**Table 5**

| No | R¹ | R⁴ | R¹⁰ | R¹¹ | R¹² | R¹³ | Salt | LCMS (M+H) |
|---|---|---|---|---|---|---|---|---|
| 5.1 | 4-imidazolyl | H | H | H | H | H | -- | 255 |
| 5.2 | 2-(1-methylimidazolyl) | H | H | H | H | H | -- | 269 |
| 5.3 | 2-(1-methylimidazolyl) | H | H | H | H | H | HCl | 269 |
| 5.4 | 4-(1-methylimidazolyl) | H | SO₂N-(CH₂CH₃)₂ | H | H | H | -- | 404 |
| 5.5 | 4-(1-methylimidazolyl) | H | F | H | H | H | -- | 287 |
| 5.6 | 4-(1-methylimidazolyl) | H | H | H | H | H | -- | 269 |
| 5.7 | 2-(5-C(CH₃)₃)-oxazolyl | H | H | H | H | H | -- | 312 |
| 5.8 | 3-(5-CH₃)-isoxazolyl | H | H | H | H | H | -- | 270 |
| 5.9 | 3-(5-CH₂OH)-isoxazolyl | H | H | H | H | H | -- | 286 |
| 5.10 | 3-(5-C₆H₅)-isoxazolyl | H | H | H | H | H | -- | 332 |
| 5.11 | 3-benzoisothiazolyl | H | H | H | H | H | -- | 322 |
| 5.12 | 2-(5-CH₃)thiadiazolyl | H | H | H | H | H | -- | 287 |
| 5.13 | 2-pyrazinyl | H | H | H | H | H | -- | 267 |
| 5.14 | 2-pyrazinyl | H | F | F | F | F | -- | 339 |
| 5.15 | 2-pyrazinyl | H | H | -C₆ | H₄- | H | -- | 317 |
| 5.16 | 2-(5-methylpyrazinyl) | H | H | H | H | H | -- | 281 |
| 5.17 | 2-pyridyl | (CO)CH₃ | H | H | H | H | -- | 308 |
| 5.18 | 3-pyridinyl | H | H | H | H | H | -- | 266 |
| 5.19 | 4-pyridinyl | H | H | H | H | H | -- | 266 |
| 5.20 | 6-(imidazolopyridinyl) | H | H | H | H | H | -- | 305 |
| 5.21 | 2-thiophenyl | H | H | H | H | H | -- | 271 |
| 5.22 | 3-isoquinolinyl | (CO)CH₃ | H | H | H | H | -- | 358 |

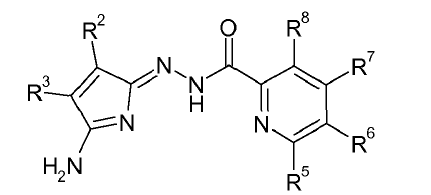

I with R¹ = A1; R⁴ = H

**Table 6**

| No. | R² and R³ together form | R⁵ | R⁶ | R⁷ | R⁸ | Salt | LCMS (M+H) |
|---|---|---|---|---|---|---|---|
| 6.1 | 3,4-thienyl | H | H | H | H | -- | 272 |
| 6.2 | 5,6-dimethy-2,3-pyrazinyl | H | H | H | H | -- | 296 |
| 6.3 | 2,3-pyrazinyl | H | H | H | H | -- | 268 |

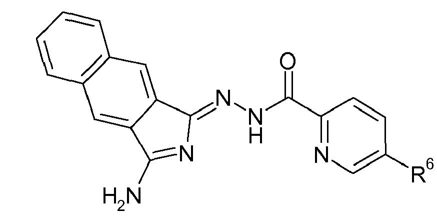

I with R¹ = A1 where R⁵, R⁷, R⁸ = H; R² + R³ form B3 R⁴ with R¹⁰, R¹³, R¹⁴, R¹⁵, R¹⁶, R¹⁷ = H; R⁴=H

**Table 7**

| No | R⁶ | Salt | LCMS (M+H) |
|---|---|---|---|
| 7.1 | H | -- | 316 |

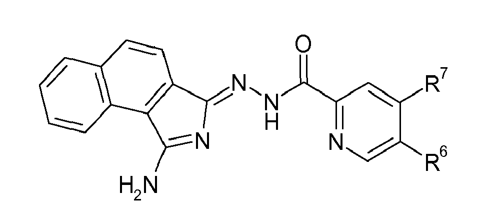

I with R¹ = A1 where R⁵ and R⁸ = H; R² + R³ form B4 where R¹² to R¹⁷ = H; R⁴ = H

**Table 8**

| No | R⁶ | R⁷ | Salt | LCMS (M+H) |
|---|---|---|---|---|
| 8.1 | H | H | -- | 316 |
| 8.2 | H | H | HCl | 316 |
| 8.3 | CH₃ | H | -- | 330 |
| 8.4 | CH₃ | H | HCl | 330 |
| 8.5 | CH₂CH₂CH₂CH₃ | H | -- | 372 |
| 8.6 | Cl | H | -- | 350 |
| 8.7 | H | Cl | -- | 350 |
| 8.8 | H | OCH₃ | -- | 346 |
| 8.9 | H | N(CH₃)₂ | -- | 359 |
| 8.10 | H | N(CH₃)₂ | HCl | 359 |
| 8.11 | H | morpholinyl | -- | 401 |
| 8.12 | H | C₆H₅ | -- | 392 |
| 8.13 | -C₆H₄- | | -- | 366 |

### Use examples

The herbicidal activity of the acylhydrazides of the formula I was demonstrated by the following greenhouse experiments:
The culture containers used were plastic flowerpots containing loamy sand with approximately 3.0% of humus as the substrate. The seeds of the test plants were sown separately for each species.

For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this has been impaired by the active ingredients.

For the post-emergence treatment, the test plants were first grown to a height of 2 to 5 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.

Depending on the species, the plants were kept at 10 - 25°C or 20 - 35°C. The test period extended over 8 to 9 days. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A good herbicidal activity is given at values of at least 70 and a very good herbicidal activity is given at values of at least 85.

The plants used in the greenhouse experiments belonged to the following species:

| Bayer Code | Scientific Name | Common Name |
|---|---|---|
| MATIN | Matricaria inodora | False chamomile |
| AGSST | Agrostis stolonifera | Creeping bentgrass |

At an application rate of 2 kg/ha, the compounds 2.16, 2.24, 2.27, 2.34, 2.37, 2.39, 2.43, 2.49, 3.9, 3.14, 3.20, 6.3 and 8.3 applied by the post-emergence method, showed very good herbicidal activity against MATIN. Compound 3.11 showed good activity on AGSST.

At an application rate of 2 kg/ha, the compound 2.43 applied by the pre-emergence method, showed good herbicidal activity against MATIN. Compound 5.14 showed good activity against AGSST.

The pharmaceutical, especially the antimalarial activity of the acylhydrazides of the formula I was demonstrated by the following in vitro experiments:
For the determination of the activity of the compounds against Plasmodium falciparum strain NF54 in human erythrocytes the [G-³H]-hypoxantine uptake assay was performed as described in Desjardins, R. E., Canfield, C. J., Haynes, J. D. & Chulay, J. D.
Quantitative assessment of antimalarial activity in vitro by a semiautomated microdilution technique. Antimicrob. Agents Chemother. 16, 710-718 (1979).

The following compounds showed very good activity in this assay (IC50<100ng/ml):
The following compounds showed good activity in this assay (IC50<1000ng/ml): For the activity of the acylhydrazides of the formula I against Mycobacteria an assay described in Hawkins, J.E., Wallace Jr., R.J., Brown, A.; 1991, Antibacterial susceptibility test: Mycobacteria: in A. Balows,W. et al. Manual of Clinical Microbiology, 5th edn., American Society of Microbiology, Washington D.C., has been used.

## Claims

1. A method of controlling undesired vegetation, which comprises allowing a
herbicidal active amount of at least one acylhydrazide of formula I 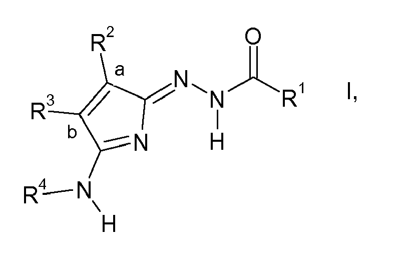 wherein the variables are as defined below:
R¹ is a five- or six-membered monocyclic or eight- to ten-membered bicyclic
heteroaryl having one to three nitrogen atoms, one or two nitrogen atoms and one sulfur atom, one nitrogen and one oxygen atom, one oxygen atom, or one sulfur atom,
which may be partially or fully halogenated and/or may be substituted by one to three substituents selected from the group consisting of cyano, nitro, C₁-C₆-alkyl, C₃-C₆-cycloalkyl, C₁-C₆-haloalkyl, C₂-C₆-alkenyl, C₂-C₆-alkynyl, hydroxy, C₁-C₆-alkoxy, C₃-C₆-cycloalkoxy, C₁-C₆-haloalkoxy, C₃-C₆-alkenyloxy, C₃-C₆-alkynyloxy, C₁-C₆-alkylthio, C₁-C₆-alkylsulfinyl, C₁-C₆-alkylsulfonyl, amino, C₁-C₆-alkylamino, di(C₁-C₆)alkylamino, C₁-C₆-alkylsulfonylamino, N(C₁-C₆-alkylsulfonyl)-N(C₁-C₆-alkyl)amino, five- or six-membered heterocyclyl having one or two nitrogen atoms, one nitrogen and one oxygen atom or one nitrogen and one sulfur atom, or phenyl,
which itself may be partially or fully halogenated and/or may be substituted by one to three substituents selected from of the group consisting of C₁-C₆-alkyl, C₁-C₆-haloalkyl and C₁-C₆-alkoxy;
R² and R³ together with the carbon atoms (a) and (b) to which they are attached
form a five- or six-membered monocyclic or eight- to ten-membered bicyclic cyclus,
which is partially unsaturated or aromatic,
which may contain one to three nitrogen atoms, one or two nitrogen
atoms and one sulfur atom, one nitrogen and one oxygen atom, one or two sulfur atoms, or one or two oxygen atoms; and which may be partially or fully halogenated and/or may be substituted
by one to three substituents selected from the group consisting of cyano, nitro, C₁-C₆-alkyl, C₃-C₆-cycloalkyl, C₁-C₆-haloalkyl, C₁-C₆-hydroxyalkyl, C₁-C₆-alkoxy-C₁-C₆-alkyl, C₁-C₆-haloalkoxy-C₁-C₆-alkyl, amino-C₁-C₆-alkyl, (C₁-C₆-alkyl)amino-C₁-C₆-alkyl, di(C₁-C₆-alkyl)amino-C₁-C₆-alkyl, C₂-C₆-alkenyl, C₂-C₆-haloalkenyl, C₂-C₆-hydroxyalkenyl, C₁-C₆-alkoxy-C₂-C₆-alkenyl, C₁-C₆-haloalkoxy-C₂-C₆-alkenyl, amino-C₂-C₆-alkenyl, (C₁-C₆-alkyl)amino-C₂-C₆-alkenyl, di(C₁-C₆-alkyl)amino-C₂-C₆-alkenyl, C₂-C₆-alkynyl, C₂-C₆-haloalkynyl, C₂-C₆-hydroxyalkynyl, C₁-C₆-alkoxy-C₂-C₆-alkynyl, C₁-C₆-haloalkoxy-C₂-C₆-alkynyl, amino-C₂-C₆-alkynyl, (C₁-C₆-alkyl)amino-C₂-C₆-alkynyl, di(C₁-C₆-alkyl)-amino-C₂-C₆-alkynyl, C₁-C₆-alkylcarbonyl, hydroxycarbonyl, C₁-C₆-alkoxycarbonyl, aminocarbonyl, (C₁-C₆-alkyl)aminocarbonyl, di(C₁-C₆-alkyl)aminocarbonyl, hydroxy, C₁-C₆-alkoxy, C₁-C₆-haloalkoxy, C₃-C₆-cycloalkoxy, C₃-C₆-alkenyloxy, C₃-C₆-alkynyloxy, C₁-C₆-alkylcarbonyloxy, C₁-C₆-hydroxyalkoxy, C₁-C₆-alkoxy-C₁-C₆-alkoxy, C₁-C₆-haloalkoxy-C₁-C₆alkoxy, amino-C₁-C₆-alkoxy, (C₁-C₆-alkyl)amino-C₁-C₆-alkoxy, di(C₁-C₆-alkyl)-amino-C₁-C₆-alkoxy, C₁-C₆-alkylsulfonyloxy, (C₁-C₆-alkyl)aminocarbonyl(C₁-C₆-alkyl)oxy, di(C₁-C₆-alkyl)aminocarbonyloxy, C₁-C₆-alkylthio, C₃-C₆-cycloalkylthio, C₃-C₆-alkenylthio, C₃-C₆-alkynylthio, C₁-C₆-hydroxyalkylthio, C₁-C₆-alkoxy-C₁-C₆-alkylthio, C₁-C₆-haloalkoxy-C₁-C₆-alkylthio, amino-C₁-C₆-alkylthio, (C₁-C₆-alkyl)amino-C₁-C₆-alkylthio, di(C₁-C₆-alkyl)amino-C₁-C₆-alkylthio, amino, C₁-C₆-alkylamino, di-(C₁-C₆-)alkylamino, C₃-C₆-cycloalkylamino, C₃-C₆-alkenylamino, C₃-C₆-alkynylamino, C₁-C₆-hydroxyalkylamino, C₁-C₆-alkoxy-C₁-C₆-alkylamino, C₁-C₆-haloalkoxy-C₁-C₆-alkylamino, amino-C₁-C₆-alkylamino, (C₁-C₆-alkyl)amino-C₁-C₆-alkylamino, di(C₁-C₆-alkyl)amino-C₁-C₆-alkylamino, C₁-C₆-alkylsulfonylamino, C₁-C₆-alkylsulfonyl(C₁-C₆-alkyl)amino, (C₁-C₆-alkyl)aminosulfonylamino, di(C₁-C₆-alkyl)-aminosulfonylamino, formylamino, formyl(C₁-C₆-alkyl)amino, (C₁-C₆-alkyl)carbonylamino, N(C₁-C₆-alkylcarbonyl)-N(C₁-C₆-alkyl)amino, (C₁-C₆-alkyl)aminocarbonylamino, di(C₁-C₆-alkyl)aminocarbonylamino, (C₁-C₆-alkyl)aminocarbonyl(C₁-C₆-alkyl)amino, C₁-C₆-alkylsulfinyl, C₁-C₆-alkylsulfonyl, C₃-C₆-cycloalkylsulfonyl, C₃-C₆-alkenylsulfonyl, C₃-C₆-alkynylsulfonyl, C₁-C₆-hydroxyalkylsulfonyl, C₁-C₆-alkoxy-C₁-C₆-alkylsulfonyl, C₁-C₆-haloalkoxy-C₁-C₆-alkylsulfonyl , amino-C₁-C₆-alkylsulfonyl, (C₁-C₆-alkyl)amino-C₁-C₆-alkylsulfonyl, di(C₁-C₆-alkyl)amino-C₁-C₆-alkylsulfonyl, aminosulfonyl, (C₁-C₆-alkyl)aminosulfonyl, di(C₁-C₆-alkyl)aminosulfonyl, phenyl, phenylcarbonyl, phenyloxy, phenylcarbonyloxy, phenylthio, phenylsulfinyl, phenylsulfonyl, phenylsulfonyloxy, phenylamino, N(phenyl)-N(C₁-C₆-alkyl)amino, phenylcarbonylamino, N-phenylcarbonyl-N(C₁-C₆-alkyl)amino,
wherein each of the phenyl groups may be partially or fully halogenated and/or may carry one to three substituents selected from the group consisting of cyano, C₁-C₆-alkyl, C₁-C₆-haloalkyl, hydroxy, C₁-C₆-alkoxy, C₁-C₆-alkylcarbonyloxy, C₁-C₆-haloalkoxy, C₃-C₆-alkenyloxy, C₃-C₆-alkynyloxy, C₁-C₆-alkylthio, C₁-C₆-alkylsulfonyl;
heterocyclyl, heterocyclyl-C₁-C₆-alkyl, heterocyclyl-C₂-C₆-alkenyl, heterocyclyl-C₂-C₆-alkynyl, heterocyclyl-C₁-C₆-alkoxy, heterocyclyl-C₁-C₆-alkylthio, heterocyclyl-C₁-C₆-alkylamino, heterocyclyl-C₁-C₆-alkylsulfonyl,heterocyclyloxy, heterocyclylthio, heterocyclylsulfinyl, heterocyclylsulfonyl, heterocyclylsulfonyloxy, heterocyclylcarbonyl, heterocyclylcarbonyloxy, heterocyclylamino, heterocyclyl-[(C₁-C₆-alkyl)amino], heterocyclylcarbonylamino, heterocyclylcarbonyl[(C₁-C₆-alkyl)amino],
wherein each of the heterocyclyl groups being three- to seven-membered and having one to three nitrogen atoms, one or two nitrogen atoms and one sulfur atom, one nitrogen atom and one oxygen atom, one oxygen atoms, one or two sulfur atoms, one oxygen atom, or one sulfur atom, and
wherein each of the heterocyclyl groups may be partially or fully
halogenated and/or may be substituted with one to three substituents selected from the group consisting of cyano, C₁-C₆-alkyl, C₁-C₆-haloalkyl, hydroxy, mercapto, C₁-C₆-alkoxy, C₁-C₆-alkylcarbonyloxy, C₁-C₆-haloalkoxy, C₃-C₆-alkenyloxy, C₃-C₆-alkynyloxy, C₁-C₆-alkylthio, C₁-C₆-alkylsulfonyl;
and
R⁴ is hydrogen, formyl or C₁-C₆-alkylcarbonyl;
including its salts;
to act on plants, their environment or on seed.

2. A method as claimed in claim 1 wherein the salts are agriculturally useful salts.

3. A herbicidal composition comprising a herbicidal active amount of at least one acylhydrazide of formula I as claimed in claim 1 and at least one inert liquid and/or solid carrier and, if appropriate, at least one surface-active substance.

4. A process for the preparation of herbicidal active compositions, which comprises mixing a herbicidal active amount of at least one acylhydrazide of formula I as claimed in claim 1 and at least one inert liquid and/or solid carrier and optionally at least one surface-active substance.

5. An acylhydrazide of formula I as defined in claim 1, including its pharmaceutically acceptable salts, for use as pharmaceutical active substance.

6. An acylhydrazide of formula I as defined in claim 5, including its pharmaceutically acceptable salts, for treating or preventing infections.

7. Use of an acylhydrazide of formula I as defined in claim 1, including its pharmaceutically acceptable salts, for the preparation of a medicament for the therapeutic and/or prophylactic treatment of infections.

8. The use as claimed in claim 7, wherein the infection is a parasitic and/or bacterial infection.

9. A pharmaceutical composition comprising a pharmaceutical effective amount of at least one acylhydrazide of formula I as claimed in claim 1, including its pharmaceutically acceptable salts, and at least one auxiliary customary for formulating pharmaceuticals.

10. A process for the preparation of a pharmaceutical composition as claimed in claim 5, which comprises mixing a pharmaceutical effective amount of at least one acylhydrazide of formula I as claimed in claim 1 and at least one pharmaceutically acceptable auxiliary.

11. Acylhydrazides of formula IA, including their agriculturally useful as well as pharmaceutically acceptable salts, which correspond to acylhydrazides of formula I as defined in claim 1, wherein in case R² and R³ together with the carbon atoms (a) and (b) to which they are attached form a six-membered monocyclic cycle, said six-membered monocyclic cycle is partially or fully halogenated and/or is substituted by one to three substituents selected from the group as defined in claim 1.

12. Process for the preparation of compounds of formula I wherein a cyclyl-1,2-dinitrile of formula II 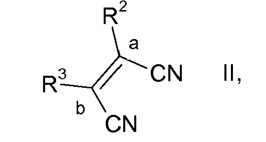 wherein R² and R³ are as defined in claim1,
is cylizised with an amine of formula IV,
NH₂R⁴ IV,
wherein R⁴ is as defined in claim1,
to give the corresponding 3-imino-3H-isoindol-1-ylamine of formula V, 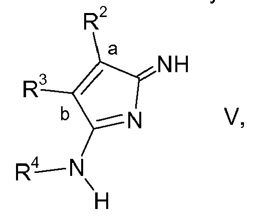 wherein R², R³ and R⁴ are as defined in claim1,
which is then reacted with the corresponding hydrazide of formula III 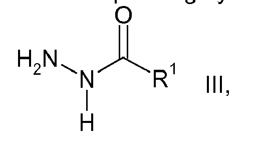 wherein R¹ is as defined in claim1.
